# EUROPEAN PATENT SPECIFICATION

(11) **EP 1 675 823 B1**
(45) Date of publication and mention of the grant of the patent: **23.07.2008**
(21) Application number: 04790747.2
(22) Date of filing: 20.10.2004
(51) Int. Cl.: C07C 311/08, C07C 235/42, C07C 217/54, C07D 213/65, A61P 11/08, A61K 31/166, A61K 31/18, A61K 31/44

(54) **PHENETHANOLAMINE DERIVATIVES FOR THE TREATMENT OF RESPIRATORY DISEASES**
PHENÄTHANOLAMINDERIVATE ZUR BEHANDLUNG VON KRANKHEITEN DER ATEMWEGE
DERIVES DE PHENETHANOLAMINE UTILISES DANS LE TRAITEMENT DE MALADIES RESPIRATOIRES

(30) Priority: 22.10.2003 GB 0324654
(43) Date of publication of application: 05.07.2006
(73) Proprietor: GLAXO GROUP LIMITED, Greenford, Middlesex UB6 0NN (GB)
(72) Inventor: BOX, Philip, Charles GlaxoSmithKline, Stevenage Hertfordshire SG1 2NY (GB); COE, Diane, Mary GlaxoSmithKline, Stevenage Hertfordshire SG1 2NY (GB); HOBBS, Heather GlaxoSmithKline, Stevenage Hertfordshire SG1 2NY (GB)
(74) Representative: Florence, Julia Anne
(86) International application number: PCT/EP2004/011952
(87) International publication number: WO 2005/040103

(56) References cited:
- EP-A1- 0 198 412
- EP-A1- 0 239 815
- WO-A1-03/091204
- US-A- 4 992 474
- ALIKHANI, VAHID ET AL: "Long-chain formoterol analogues: an investigation into the effect of increasing amino-substituent chain length on the .beta.2-adrenoceptor activity" BIOORGANIC & MEDICINAL CHEMISTRY LETTERS , 14(18), 4705-4710 CODEN: BMCLE8; ISSN: 0960-894X, 2004, XP002323505

## Description

The present invention is concerned with phenethanolamine derivatives, processes for their preparation, compositions containing them and their use in medicine, particularly in the prophylaxis and treatment of respiratory diseases.

EP0 239 815 describes substituted thiazole and oxazole compounds useful in the treatment of diabetes mellitus and obesity. EP0 198 412 describes phenethanolamine derivatives and their pharmaceutically compatible salts which can be used for the treatment of obesity and of diabetes mellitus or of conditions which are associated with an increased protein breakdown, or as a feed additive for fattening animals. US4,992,474 describes phenethanolamine derivatives and physiologically acceptable salts and solvates thereof which have a selective stimulant action at β₂-adrenoreceptors and may be used *inter alia* in the treatment of diseases associated with reversible airways obstructions such as asthma and chronic bronchitis. WO03/091204 relates to novel phenethanolamine derivatives and their use in therapy, in particular their use in the prophylaxis and treatment of respiratory diseases. Alikhani, V. et al, Bioorganic & Medicinal Chemistry Letters, 14 (2004) 4705-4710, describe an investigation into the effect of increasing amino-substituent chain length on the β₂-adrenoreceptor activity of long-chain formoterol analogues.

Certain phenethanolamine compounds are known in the art as having selective stimulant action at β₂-adrenoreceptors and therefore having utility in the treatment of bronchial asthma and related disorders. Thus GB 2 140 800 describes phenethanolamine compounds including 4-hydroxy-α¹-[[[6-(4-phenylbutoxy)hexyl]amino]methyl]-1,3-benzenedimethanol 1-hydroxy-2-naphthalenecarboxylate (salmeterol xinafoate) which is now used clinically in the treatment of such medical conditions.

Although salmeterol and the other commercially available β₂-adrenoreceptor agonists are effective bronchodilators, the duration of action is approximately 12 hours, hence twice daily dosing is often required. There is therefore a clinical need for compounds having potent and selective stimulant action at β₂-adrenoreceptors and having an advantageous profile of action.

According to the present invention, there is provided a compound of formula (I) 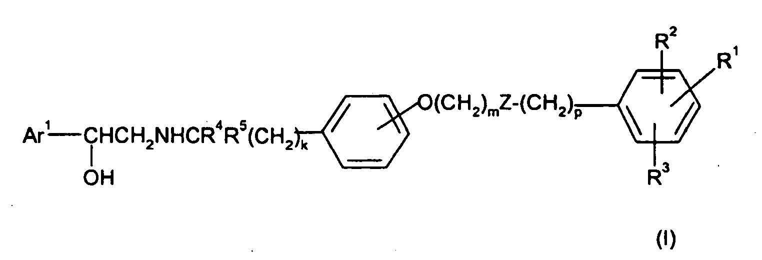 or a salt or solvate thereof, wherein:
k is an integer of from 1 to 3;
m is an integer of from 2 to 4;
p is an integer of from 1 to 4, preferably 2;
Z is O or CH₂-

R¹ is selected from hydrogen, C₁₋₆alkyl, hydroxy, C₁₋₆alkoxy, cyano, nitro, halo, C₁₋₆haloalkyl, XCO₂R⁸, -XC(O)NR⁷R⁸, -XNR⁶C(O)R⁷, -XNR⁶C(O)NR⁷R⁸, -XNR⁶C(O)NC(O)NR⁷R⁸, -XNR⁶SO₂R⁷, -XSO₂NR⁹R¹⁰, XSR⁶, XSOR⁶, XSO₂R⁶, XNR⁶SO₂NR⁷R⁸, XNR⁶SO₂NR⁷COOR⁷, -XNR⁷R⁸, -XNR⁶C(O)OR⁷,
or R¹ is selected from -X-aryl, -X-hetaryl, or -X-(aryloxy), each optionally substituted by 1 or 2 groups independently selected from hydroxy, C₁₋₆alkoxy, halo, C₁₋₆alkyl,
C₁₋₆haloalkyl, -NR⁶C(O)R⁷, SR⁶, SOR⁶, -SO₂R⁶, -SO₂NR⁹R¹⁰, -CO₂R⁸, -N R⁷R⁸, or hetaryl optionally substituted by 1 or 2 groups independently selected from hydroxy, C₁₋₆alkoxy, halo, C₁₋₆alkyl, or C₁₋₆haloalkyl;
X is -(CH₂)_{q}- or C₂₋₆ alkenylene;
q is an integer from 0 to 6, preferably 0 to 4;
R⁶ and R⁷ are independently selected from hydrogen, C₁₋₆alkyl, C₃₋₇cycloalkyl, aryl, hetaryl, hetaryl(C₁₋₆alkyl)- and aryl(C₁₋₆alkyl)- and R⁶ and R⁷ are each independently optionally substituted by 1 or 2 groups independently selected from halo, C₁₋₆alkyl, C₃₋₇ cycloalkyl, C₁₋₆ alkoxy, C₁₋₆haloalkyl, -NHC(O)(C₁₋₆alkyl), -SO₂(C₁₋₆alkyl), -SO₂(aryl), -CO₂H, -CO₂(C₁₋₄alkyl), -NH₂, -NH(C₁₋₆alkyl), aryl(C₁₋₆alkyl)-, aryl(C₂₋₆alkenyl)-, aryl(C₂₋₆alkynyl)-, hetaryl(C₁₋₆alkyl)-, -NHSO₂aryl, -NH(hetarylC₁₋₆alkyl), -NHSO₂hetaryl, -NHSO₂(C₁₋₆alkyl), -NHC(O)aryl, or -NHC(O)hetaryl:
R⁸ is selected from hydrogen, C₁₋₆alkyl and C₃₋₇ cycloalkyl;
or R⁷ and R⁸, together with the nitrogen atom to which they are bonded, form a 5-, 6- or 7-membered nitrogen - containing ring;
R⁹ and R¹⁰ are independently selected from hydrogen, C₁₋₆alkyl, C₃₋₇cycloalkyl, aryl, hetaryl, hetaryl(C₁₋₆alkyl)- and aryl(C₁₋₆alkyl)-, or R⁹ and R¹⁰, together with the nitrogen to which they are bonded, form a 5-, 6-, or 7- membered nitrogen containing ring;
and R⁹ and R¹⁰ are each optionally substituted by one or two groups independently selected from halo, C₁₋₆alkyl, C₃₋₇cycloalkyl and C₁₋₆haloalkyl;
R² is selected from hydrogen, hydroxy, C₁₋₆alkyl, C₁₋₆alkoxy, halo, aryl, aryl(C₁₋₆alkyl)-, C₁₋₆haloalkoxy, and C₁₋₆haloalkyl;
R³ is selected from hydrogen, hydroxy, C₁₋₆alkyl, C₁₋₆alkoxy, halo, aryl, aryl(C₁₋₆alkyl)-,
C₁₋₆haloalkoxy, and C₁₋₆haloalkyl;
R⁴ and R⁵ are independently selected from hydrogen and C₁₋₄ alkyl with the proviso that the total number of carbon atoms in R⁴ and R⁵ is not more than 4: and
Ar¹ is a group selected from 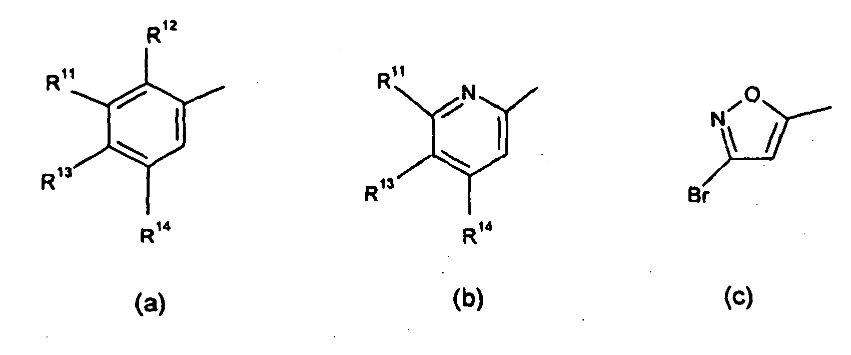 and 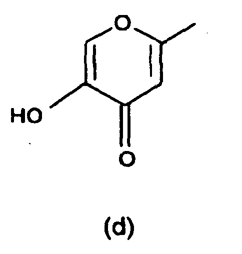 wherein R¹¹ represents hydrogen, halogen, -(CH₂)ₙOR¹⁵, -NR¹⁵C(O)R¹⁶, -NR¹⁵SO₂R¹⁶, -SO₂NR¹⁵R¹⁶, -NR¹⁵R¹⁶, -OC(O)R¹⁷ or OC(O)NR¹⁵R¹⁶,
and R¹² represents hydrogen, halogen or C₁₋₄ alkyl;
or R¹¹ represents -NHR¹⁸ and R¹² and -NHR¹⁸ together form a 5- or 6- membered heterocyclic ring;
R¹³ represents hydrogen, halogen, -OR¹⁵ or -NR¹⁵R¹⁶;
R¹⁴ represents hydrogen, halogen, haloC₁₋₄ alkyl, -OR¹⁵, -NR¹⁵ R¹⁶, -OC(O)R¹⁷ or OC(O)NR¹⁵R¹⁶;
R¹⁵ and R¹⁸ each independently represents hydrogen or C₁₋₄ alkyl, or in the groups -NR¹⁵R¹⁶, -SO₂NR¹⁵R¹⁶ and -OC(O)NR¹⁵R¹⁶, R¹⁵ and R¹⁶ independently represent hydrogen or C₁₋₄ alkyl or together with the nitrogen atom to which they are attached form a 5-, 6- or 7- membered nitrogen-containing ring,
R¹⁷ represents an aryl (eg phenyl or naphthyl) group which may be unsubstituted or substituted by one or more substituents selected from halogen, C₁₋₄ alkyl, hydroxy, C₁₋₄ alkoxy or halo C₁₋₄ alkyl; and
n is zero or an integer from 1 to 4;
provided that in the group (a), when R¹¹ represents -(CH₂)ₙOR¹⁵ and n is 1, R¹³ is not OH.
with the proviso that when one of R⁴ and R⁵ is hydrogen and the other is hydrogen or methyl, k is 1 or 2, Z is oxygen, the moiety -Ph(R¹)(R²)(R³) is phenyl, p-fluorophenyl or p-phenoxyphenyl then ring (a) is not phenyl monosubstituted in the m-position by bromine, chlorine or fluorine, or unsubstituted phenyl.

In the compound of formula (I), the group R¹ is preferably attached to the para- or meta-position, and more preferably to the meta-position relative to the -Z(CH₂)ₚ link.

In the compounds of formula (I), the group R¹ is suitably selected from hydrogen,
C₁₋₄alkyl, hydroxy, cyano, C₁₋₆alkoxy, halo,
XCO₂R⁶, XNR⁶COR⁷, XCONR⁷R⁸, -NR⁶C(O)NR⁷R⁸,
XSOR⁶, XNR⁶SO₂NR⁷R⁸, XNR⁸SO₂NR⁷CO₂R⁷ and -NR⁶SO₂R⁷
wherein R⁶ and R⁷ are as defined above or more suitably wherein R⁶ is hydrogen and R⁷ is selected from hydrogen, C₁₋₆alkyl, C₃₋₆cycloalkyl, and aryl and is optionally substituted as described above.

R¹ may be for example hydrogen or -XC(O)NR⁷R⁸.

Where R¹ is -XNR⁶C(O)NR⁷R⁸, R⁶ and R⁷ may, together with the -NC(O)N- portion of the group R¹ to which they are bonded, form a saturated or unsaturated ring, preferably a 5-, 6-, or 7- membered ring, for example an imidazolidine ring, such as imidazolidine-2,4-dione.

Where R¹ is -XNR⁶C(O)OR⁷, R⁶ and R⁷ may, together with the -NC(O)O- portion of the group R¹ to which they are bonded, form a saturated or unsaturated ring, preferably a 5-, 6-, or 7- membered ring, for example an oxazolidine ring, such as oxazolidine-2,4-dione.

Where R¹ is -XC(O)NR⁷R⁸ or -XNR⁶C(O)NR⁷R⁸, R⁷ and R⁸ may, together with the nitrogen to which they are bonded, form a 5-, 6-, or 7- membered nitrogen containing ring.

In the compounds of formula (I) wherein the group R¹ is substituted by R⁶ and/or R⁸, R⁶ and/or R⁸ are suitably hydrogen.

In the compounds of formula (I), R⁴ and R⁵ are preferably independently selected from hydrogen and methyl, more preferably R⁴ and R⁵ are both hydrogen.

In the compounds of formula (I) R² and R³ preferably each represent hydrogen.

Preferably the moiety 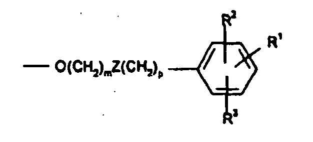 is attached to the para position of the 'central' phenyl ring, relative to the -NHCR⁴R⁵(CH₂)ₖ- moiety.

In the compounds of formula (I) the group Ar¹ is preferably selected from groups (a) and (b) above. In said groups (a) and (b), when R¹¹ represents halogen this is preferably chlorine or fluorine. R¹⁵ and R¹⁶ preferably each independently represent hydrogen or methyl. R¹⁷ preferably represents substituted phenyl. The integer n preferably represents zero or 1. Thus for example -(CH₂)ₙOR¹⁵ preferably represents OH or -CH₂OH;
NR¹⁵C(O)R¹⁶ preferably represents -NHC(O)H;
-SO₂NR¹⁵R¹⁶ preferably represents -SO₂NH₂ or SO₂NHCH₃;
NR¹⁵R¹⁶ preferably represents -NH₂;
-OC(O)R¹⁷ preferably represents substituted benzoyloxy eg. OC(O)-C₆H₄-(p-CH₃); and
-OC(O)N R¹⁵ R¹⁶ preferably represents OC(O)N(CH₃)₂.

When R¹¹ represents NHR¹⁸ and together with R¹² forms a 5- or 6- membered heterocyclic ring -NHR¹⁸-R¹²- preferably represents a group:
-NH-CO-R¹⁹- where R¹⁹ is an alkyl, alkenyl or alkyloxy group;
-NH-SO₂R²⁰- where R²⁰ is an alkyloxy group;
-NH-R²¹- where R²¹ is an alkyl or alkenyl group optionally substituted by COOR²² where
R²² is C₁₋₄ alkyl; or
-NH-CO-S-;
wherein said alkyl, and alkenyl groups and moieties contain 1 or 2 carbon atoms.

Particularly preferred groups (a) and (b) may be selected from the following groups (i) to (xx): 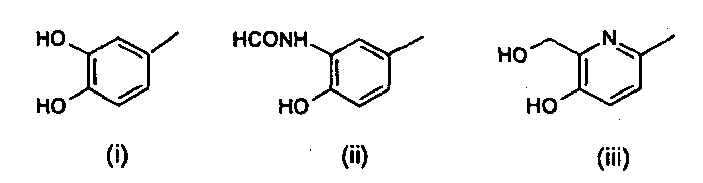 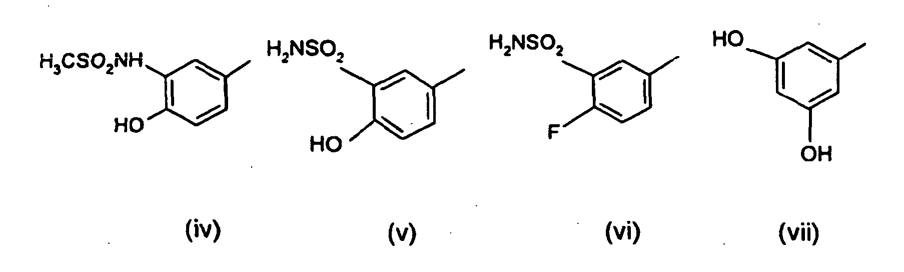 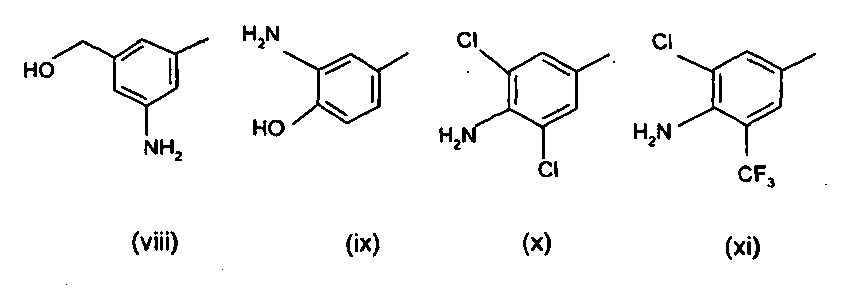 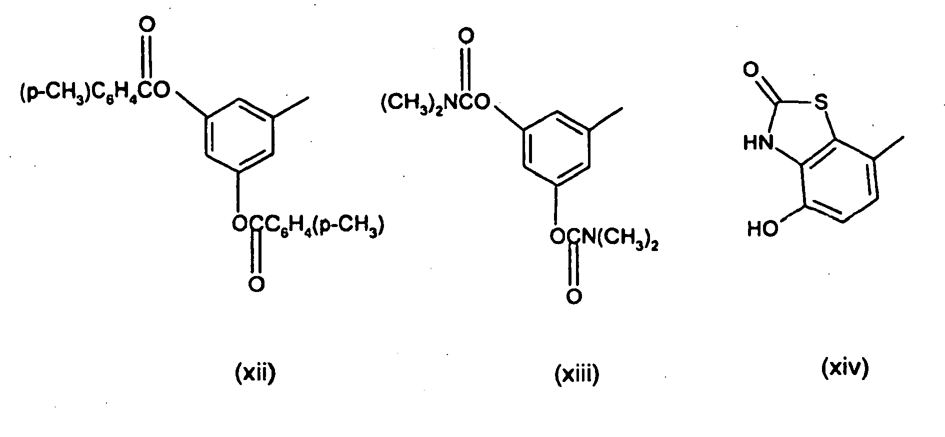 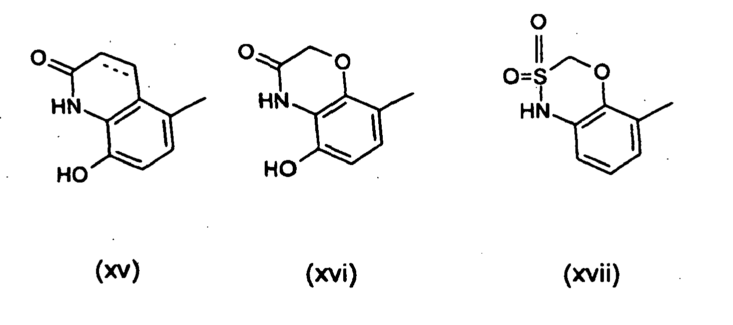 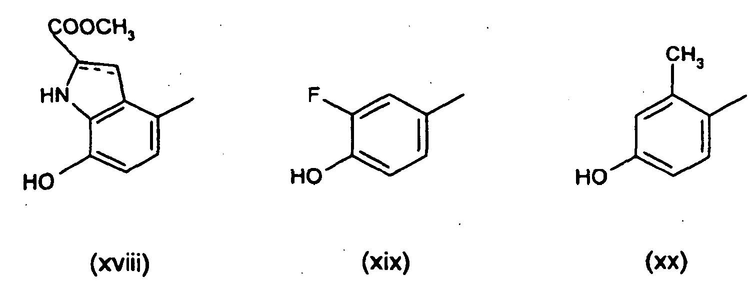 wherein the dotted line in (xv) and (xviii) denotes an optional double bond.

Suitably (a) and (b) may be selected from a group of structure (iii), (iv) or (xix).

In the compounds of formula (I) an aryl group or moiety may be for example phenyl or naphthyl.

In the compounds of formula (I) hetaryl group may be for example pyrrolyl, furyl, thienyl, pyridinyl, pyrazinyl, pyridazinyl, imidazolyl, tetrazolyl, tetrahydrofuranyl, oxazolyl, thiazolyl or thiadiazolyl.

It is to be understood that the present invention covers all combinations of particular and preferred groups described hereinabove.

The compounds of formula (I) include an asymmetric centre, namely the carbon atom of the 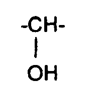 group. The present invention includes both (S) and (R) enantiomers either in substantially pure form or admixed in any proportions. Preferably, the compounds of the invention are in the form of the (R) enantiomers.

Similarly, where R⁴ and R⁵ are different groups, the carbon atom to which they are attached will be an asymmetric centre and the present invention includes both (S) and (R) enantiomers at this centre either in substantially pure form or admixed in any proportions.

Thus the compounds of formula (I) include all enantiomers and diastereoisomers as well as mixtures thereof in any proportions.

Preferred compounds of the invention include:
3-{[2-(4-{2-[((2*R*)-2-hydroxy-2-{4-hydroxy-3-[(methylsulfonyl)amino]phenyl}ethyl)amino]ethyl}phenoxy)ethoxy]methyl}benzamide;
*N*-{2-hydroxy-5-[(1*R*)-1-hydroxy-2-({2-[4-(4-phenylbutoxy)phenyl]ethyl}amino)ethyl]phenyl}methanesulfonamide;
*N*-(5-{(1*R*)-2-[(2-{4-[2-(benzyloxy)ethoxy]phenyl}ethyl)amino]-1-hydroxyethyl}-2-hydroxyphenyl)methanesulfonamide;
3-({2-[4-(2-{[(2*R*)-2-(3-fluoro-4-hydroxyphenyl)-2-hydroxyethyl]amino}ethyl)phenoxy]ethoxy}methyl)benzamide;
4-{(1*R*)-2-[(2-{4-[2-(benzyloxy)ethoxy]phenyl}ethyl)amino]-1-hydroxyethyl}-2-fluorophenol;
2-fluoro-4-[(1*R*)-1-hydroxy-2-({2-[4-(4-phenylbutoxy)phenyl]ethyl}amino)ethyl]phenol;
3-[(2-{4-[2-({2-hydroxy-2-[5-hydroxy-6-(hydroxymethyl)pyridin-2-yl]ethyl}amino)ethyl]phenoxy}ethoxy)methyl]benzamide;
6-{2-[(2-{4-[2-(benzyloxy)ethoxy]phenyl}ethyl)amino]-1-hydroxyethyl}-2-(hydroxymethyl)pyridin-3-ol;
2-(hydroxymethyl)-6-[1-hydroxy-2-({2-(4-(4-phenylbutoxy)phenyl]ethyl}amino)ethyl]pyridin-3-ol;
and salts and solvates thereof.

Salts and solvates of compounds of formula (I) which are suitable for use in medicine are those wherein the counterion or associated solvent is pharmaceutically acceptable. However, salts and solvates having non-pharmaceutically acceptable counterions or associated solvents are within the scope of the present invention, for example, for use as intermediates in the preparation of other compounds of formula (I) and their pharmaceutically acceptable salts and solvates.

Suitable salts according to the invention include those formed with both organic and inorganic acids or bases. Pharmaceutically acceptable acid addition salts include those formed from hydrochloric, hydrobromic, sulphuric, citric, tartaric, phosphoric, lactic, pyruvic, acetic, trifluoroacetic, triphenylacetic, sulphamic, sulphanilic, succinic, oxalic, fumaric, maleic, malic, glutamic, aspartic, oxaloacetic, methanesulphonic, ethanesulphonic, arylsulphonic (for example p-toluenesulphonic, benzenesulphonic, naphthalenesulphonic or naphthalenedisulphonic), salicylic, glutaric, gluconic, tricarballylic, cinnamic, substituted cinnamic (for example, phenyl, methyl , methoxy or halo substituted cinnamic, including 4-methyl and 4-methoxycinnamic acid), ascorbic, oleic, naphthoic, hydroxynaphthoic (for example 1- or 3-hydroxy-2-naphthoic), naphthaleneacrylic (for example naphthalene-2-acrylic), benzoic, 4-methoxybenzoic, 2- or 4-hydroxybenzoic, 4-chlorobenzoic, 4-phenylbenzoic, benzeneacrylic (for example 1,4-benzenediacrylic) and isethionic acids. Pharmaceutically acceptable base salts include ammonium salts, alkali metal salts such as those of sodium and potassium, alkaline earth metal salts such as those of calcium and magnesium and salts with organic bases such as dicyclohexyl amine and N-methyl-D-glucamine.

Pharmaceutically acceptable esters of the compounds of formula (I) may have a hydroxyl group converted to a C₁₋₆alkyl, aryl, aryl C₁₋₆ alkyl, or amino acid ester.

As mentioned above, the compounds of formulae (I) are selective β₂-adrenoreceptor agonists as demonstrated using functional or reporter gene readout from cell lines transfected with human beta-adrenoreceptors as described below.

Therefore, compounds of formula (I) and their pharmaceutically acceptable salts, and solvates have use in the prophylaxis and treatment of clinical conditions for which a selective β₂-adrenoreceptor agonist is indicated. Such conditions include diseases associated with reversible airways obstruction such as asthma, chronic obstructive pulmonary diseases (COPD) (e.g. chronic and wheezy bronchitis, emphysema), respiratory tract infection and upper respiratory tract disease.

Other conditions which may be treated include premature labour, depression, congestive heart failure, skin diseases (e.g. inflammatory, allergic, psoriatic, and proliferative skin diseases), conditions where lowering peptic acidity is desirable (e.g. peptic and gastric ulceration) and muscle wasting disease.

In the alternative, there is also provided a compound of formula (I), or a pharmaceutically acceptable salt or solvate thereof for use in medical therapy, particularly, for use in the prophylaxis or treatment of a clinical condition in a mammal, such as a human, for which a selective β₂-adrenoreceptor agonist is indicated. In particular, there is provided a compound of formula (I), or a pharmaceutically acceptable salt or solvate thereof for the prophylaxis or treatment of a disease associated with reversible airways obstruction such as asthma, chronic obstructive pulmonary disease (COPD), respiratory tract infection or upper respiratory tract disease. In a further aspect, there is provided a compound of formula (I), or a pharmaceutically acceptable salt or solvate thereof for the prophylaxis or treatment of a clinical condition selected from premature labour, depression, congestive heart failure, skin diseases (e.g. inflammatory, allergic, psoriatic, and proliferative skin diseases), conditions where lowering peptic acidity is desirable (e.g. peptic and gastric ulceration) or muscle wasting disease.

The present invention also provides the use of a compound of formula (I), or a pharmaceutically acceptable salt or solvate thereof in the manufacture of a medicament for the prophylaxis or treatment of a clinical condition for which a selective β₂-adrenoreceptor agonist is indicated, for example a disease associated with reversible airways obstruction such as asthma, chronic obstructive pulmonary disease (COPD), respiratory tract infection or upper respiratory tract disease. In a further aspect, there is provided a compound of formula (I), or a pharmaceutically acceptable salt or solvate thereof in the manufacture of a medicament for the prophylaxis or treatment of a clinical condition selected from premature labour, depression, congestive heart failure, skin diseases (e.g. inflammatory, allergic, psoriatic, and proliferative skin diseases), conditions where lowering peptic acidity is desirable (e.g. peptic and gastric ulceration) and muscle wasting disease.

The amount of a compound of formula (I), or a pharmaceutically acceptable salt or solvate thereof which is required to achieve a therapeutic effect will, of course, vary with the particular compound, the route of administration, the subject under treatment, and the particular disorder or disease being treated. The compounds of the invention may be administered by inhalation at a dose of from 0.0005mg to 10 mg, preferably 0.005mg to 0.5mg, for example 0.05mg to 0.5mg per day. The dose range for adult humans is generally from 0.0005 mg to 10mg per day and preferably 0.01 mg to 1 mg per day, most preferably 0.05mg to 0.5mg per day.

While it is possible for the compound of formula (I), or a pharmaceutically acceptable salt or solvate thereof to be administered alone, it is preferable to present it as a pharmaceutical formulation.

Accordingly, the present invention further provides a pharmaceutical formulation comprising a compound of formula (I), or a pharmaceutically acceptable salt or solvate thereof, and a pharmaceutically acceptable carrier or excipient, and optionally one or more other therapeutic ingredients.

Hereinafter, the term "active ingredient" means a compound of formula (I), or a pharmaceutically acceptable salt or solvate thereof.

The formulations include those suitable for oral, parenteral (including subcutaneous, intradermal, intramuscular, intravenous and intraarticular), inhalation (including fine particle dusts or mists which may be generated by means of various types of metered dose pressurised aerosols, nebulisers or insufflators), rectal and topical (including dermal, buccal, sublingual and intraocular) administration although the most suitable route may depend upon for example the condition and disorder of the recipient. The formulations may conveniently be presented in unit dosage form and may be prepared by any of the methods well known in the art of pharmacy. All methods include the step of bringing the active ingredient into association with the carrier which constitutes one or more accessory ingredients. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example saline or water-for-injection, immediately prior to use. Extemporaneous injection solutions and suspensions may be prepared from sterile powders, granules and tablets of the kind previously described.

Dry powder compositions for topical delivery to the lung by inhalation may, for example, be presented in capsules and cartridges of for example gelatine, or blisters of for example laminated aluminium foil, for use in an inhaler or insufflator.. Powder blend formulations generally contain a powder mix for inhalation of the compound of the invention and a suitable powder base (carrier/diluent/excipient substance) such as mono-, di or polysaccharides (eg. lactose or starch). Use of lactose is preferred.

Each capsule or cartridge may generally contain between 20µg-10mg of the compound of formula (I) optionally in combination with another therapeutically active ingredient. Alternatively, the compound of the invention may be presented without excipients. Packaging of the formulation may be suitable for unit dose or multi-dose delivery. In the case of multi-dose delivery, the formulation can be pre-metered (eg as in Diskus, see GB 2242134, US Patent Nos. 6,632,666, 5,860,419, 5,873,360 and 5,590,645 or Diskhaler, see GB 2178965, 2129691 and 2169265, US Patent No.s 4,778,054, 4,811,731, 5,035,237, the disclosures of which are hereby incorporated by reference) or metered in use (eg as in Turbuhaler, see EP 69715 or in the devices described in US Patents No. 6,321,747 the disclosures of which are hereby incorporated by reference). An example of a unit-dose device is Rotahaler (see GB 2064336 and US Patent No. 4,353,656, the disclosures of which are hereby incorporated by reference). The Diskus inhalation device comprises an elongate strip formed from a base sheet having a plurality of recesses spaced along its length and a lid sheet hermetically but peelably sealed thereto to define a plurality of containers, each container having therein an inhalable formulation containing a compound of formula (I) preferably combined with lactose. Preferably, the strip is sufficiently flexible to be wound into a roll. The lid sheet and base sheet will preferably have leading end portions which are not sealed to one another and at least one of the said leading end portions is constructed to be attached to a winding means. Also, preferably the hermetic seal between the base and lid sheets extends over their whole width. The lid sheet may preferably be peeled from the base sheet in a longitudinal direction from a first end of the said base sheet.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the compound of formula (I) optionally in combination with another therapeutically active ingredient and a suitable propellant such as a fluorocarbon or hydrogen-containing chlorofluorocarbon or mixtures thereof, particularly hydrofluoroalkanes, e.g. dichlorodifluoromethane, trichlorofluoromethane, dichlorotetrafluoroethane, especially 1,1,1,2-tetrafluoroethane, 1,1,1,2,3,3,3-heptatluoro-n-propane or a mixture thereof. Carbon dioxide or other suitable gas may also be used as propellant. The aerosol composition may be excipient free or may optionally contain additional formulation excipients well known in the art such as surfactants eg oleic acid or lecithin and cosolvents eg ethanol. Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10µm, preferably 2-5µm. Particles having a size above 20µm are generally too large when inhaled to reach the small airways. To achieve these particle sizes the particles of the active ingredient as produced may be size reduced by conventional means eg by micronisation. The desired fraction may be separated out by air classification or sieving. Preferably, the particles will be crystalline. When an excipient such as lactose is employed, generally, the particle size of the excipient will be much greater than the inhaled medicament within the present invention. When the excipient is lactose it will typically be present as milled lactose, wherein not more than 85% of lactose particles will have a MMD of 60-90µm and not less than 15% will have a MMD of less than 15µm.

Intranasal sprays may be formulated with aqueous or non-aqueous vehicles with the addition of agents such as thickening agents, buffer salts or acid or alkali to adjust the pH, isotonicity adjusting agents or anti-oxidants.

Solutions for inhalation by nebulation may be formulated with an aqueous vehicle with the addition of agents such as acid or alkali, buffer salts, isotonicity adjusting agents or antimicrobials. They may be sterilised by filtration or heating in an autoclave, or presented as a non-sterile product.

Formulations for rectal administration may be presented as a suppository with the usual carriers such as cocoa butter or polyethylene glycol.
Formulations for topical administration in the mouth, for example buccally or sublingually, include lozenges comprising the active ingredient in a flavoured basis such as sucrose and acacia or tragacanth, and pastilles comprising the active ingredient in a basis such as gelatin and glycerin or sucrose an acacia.

Preferred unit dosage formulations are those containing an effective dose, as hereinbefore recited, or an appropriate fraction thereof, of the active ingredient.

It should be understood that in addition to the ingredients particularly mentioned above, the formulations of this invention may include other agents conventional in the art having regard to the type of formulation in question, for example those suitable for oral administration may include flavouring agents.

The compounds and pharmaceutical formulations according to the invention may be used in combination with or include one or more other therapeutic agents, for example selected from anti-inflammatory agents, anticholinergic agents (particularly an M₁, M₂, M₁/M₂ or M₃ receptor antagonist), other β₂-adrenoreceptor agonists, antiinfective agents (e.g. antibiotics, antivirals), or antihistamines. The invention thus provides, in a further aspect, a combination comprising a compound of formula (I) or a pharmaceutically acceptable salt or solvate thereof together with one or more other therapeutically active agents, for example selected from an anti-inflammatory agent (for example a corticosteroid or an NSAID), an anticholinergic agent, another β₂-adrenoreceptor agonist, an antiinfective agent (e.g. an antibiotic or an antiviral), or an antihistamine. Preferred are combinations comprising a compound of formula (I) or a pharmaceutically acceptable salt or solvate thereof together with a corticosteroid, and/or an anticholinergic, and/or a PDE-4 inhibitor. Preferred combinations are those comprising one or two other therapeutic agents.

It will be clear to a person skilled in the art that, where appropriate, the other therapeutic ingredient(s) may be used in the form of salts, (e.g. as alkali metal or amine salts or as acid addition salts), or prodrugs, or as esters (e.g. lower alkyl esters), or as solvates (e.g. hydrates) to optimise the activity and/or stability and/or physical characteristics (e.g. solubility) of the therapeutic ingredient. It will be clear also that where appropriate, the therapeutic ingredients may be used in optically pure form.

Suitable anti-inflammatory agents include corticosteroids and NSAIDs. Suitable corticosteroids which may be used in combination with the compounds of the invention are those oral and inhaled corticosteroids and their pro-drugs which have anti-inflammatory activity. Examples include methyl prednisolone, prednisolone, dexamethasone, fluticasone propionate, 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester, 6α,9α-difluoro-11β-hydroxy-16α-methyl-3-oxo-17α-propionyloxy- androsta-1,4-diene-17β-carbothioic acid S-(2-oxo-tetrahydro-furan-3S-yl) ester, beclomethasone esters (e.g. the 17-propionate ester or the 17,21-dipropionate ester), budesonide, flunisolide, mometasone esters (e.g. the furoate ester), triamcinolone acetonide, rofleponide, ciclesonide, butixocort propionate, RPR-106541, and ST-126. Preferred corticosteroids include fluticasone propionate, 6α,9α-difluoro-11β-hydroxy-16α-methyl-17α-[(4-methyl-1,3-thiazole-5-carbonyl)oxy]-3-oxo-androsta-1,4-diene-17β-carbothioic acid *S-*fluoromethyl ester and 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester, more preferably 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester.

Suitable NSAIDs include sodium cromoglycate, nedocromil sodium, phosphodiesterase (PDE) inhibitors (e.g. theophylline, PDE4 inhibitors or mixed PDE3/PDE4 inhibitors), leukotriene antagonists, inhibitors of leukotriene synthesis, iNOS inhibitors, tryptase and elastase inhibitors, beta-2 integrin antagonists and adenosine receptor agonists or antagonists (e.g. adenosine 2a agonists), cytokine antagonists (e.g. chemokine antagonists) or inhibitors of cytokine synthesis. Suitable other β₂-adrenoreceptor agonists include salmeterol (e.g. as the xinafoate), salbutamol (e.g. as the sulphate or the free base), formoterol (e.g. as the fumarate), fenoterol or terbutaline and salts thereof.

Of particular interest is use of the compound of formula (I) in combination with a phosphodiesterase 4 (PDE4) inhibitor or a mixed PDE3/PDE4 inhibitor. The PDE4-specific inhibitor useful in this aspect of the invention may be any compound that is known to inhibit the PDE4 enzyme or which is discovered to act as a PDE4 inhibitor, and which are only PDE4 inhibitors, not compounds which inhibit other members of the PDE family as well as PDE4. Generally it is preferred to use a PDE4 inhibitor which has an IC₅₀ ratio of about 0.1 or greater as regards the IC₅₀ for the PDE4 catalytic form which binds rolipram with a high affinity divided by the IC₅₀ for the form which binds rolipram with a low affinity. For the purposes of this disclosure, the cAMP catalytic site which binds R and S rolipram with a low affinity is denominated the "low affinity" binding site (LPDE 4) and the other form of this catalytic site which binds rolipram with a high affinity is denominated the "high affinity" binding site (HPDE 4). This term "HPDE4" should not be confused with the term "hPDE4" which is used to denote human PDE4.

A method for determining IC₅₀s ratios is set out in US patent 5,998,428 which is incorporated herein in full by reference as though set out herein. See also PCT application WO 00/51599 for another description of said assay.

The preferred PDE4 inhibitors of use in this invention will be those compounds which have a salutary therapeutic ratio, i.e., compounds which preferentially inhibit cAMP catalytic activity where the enzyme is in the form that binds rolipram with a low affinity, thereby reducing the side effects which apparently are linked to inhibiting the form which binds rolipram with a high affinity. Another way to state this is that the preferred compounds will have an IC₅₀ ratio of about 0.1 or greater as regards the IC₅₀ for the PDE4 catalytic form which binds rolipram with a high affinity divided by the IC₅₀ for the form which binds rolipram with a low affinity.

A further refinement of this standard is that of one wherein the PDE4 inhibitor has an IC₅₀ ratio of about 0.1 or greater; said ratio is the ratio of the IC₅₀ value for competing with the binding of 1nM of [³H]R-rolipram to a form of PDE4 which binds rolipram with a high affinity over the IC₅₀ value for inhibiting the PDE4 catalytic activity of a form which binds rolipram with a low affinity using 1 µM[³H]-cAMP as the substrate.

Most preferred are those PDE4 inhibitors which have an IC₅₀ ratio of greater than 0.5, and particularly those compounds having a ratio of greater than 1.0. Preferred compounds are *cis* 4-cyano-4-(3-cyclopentyloxy-4-methoxyphenyl)cyclohexan-1-carboxylic acid, 2-carbomethoxy-4-cyano-4-(3-cyclopropylmethoxy-4-difluoromethoxyphenyl)cyclohexan-1-one and *cis*-[4-cyano-4-(3-cyclopropylmethoxy-4-difluoromethoxyphenyl)cyclohexan-1-ol]; these are examples of compounds which bind preferentially to the low affinity binding site and which have an IC₅₀ ratio of 0.1 or greater.

Other compounds of interest include:

Compounds set out in U.S. patent 5,552,438 issued 03 September, 1996; this patent and the compounds it discloses are incorporated herein in full by reference. The compound of particular interest, which is disclosed in U.S. patent 5,552,438, is *cis*-4-cyano-4-[3-(cyclopentyloxy)4-methoxyphenyl]cyclohexane-1-carboxylic acid (also known as cilomalast) and its salts, esters, pro-drugs or physical forms;

AWD-12-281 from elbion (Hofgen, N. et al. 15th EFMC Int Symp Med Chem (Sept 6-10, Edinburgh) 1998, Abst P.98; CAS reference No. 247584020-9); a 9-benzyladenine derivative nominated NCS-613 (INSERM); D-4418 from Chiroscience and Schering-Plough; a benzodiazepine PDE4 inhibitor identified as CI-1018 (PD-168787) and attributed to Pfizer; a benzodioxole derivative disclosed by Kyowa Hakko in WO99/16766; K-34 from Kyowa Hakko; V-11294A from Napp (Landells, L.J. et al. Eur Resp J [Annu Cong Eur Resp Soc (Sept 19-23, Geneva) 1998] 1998, 12 (Suppl. 28): Abst P2393); roflumilast (CAS reference No 162401-32-3) and a pthalazinone (WO99/47505, the disclosure of which is hereby incorporated by reference) from Byk-Gulden; Pumafentrine, (-)-p-[(4*a*R*,10*b*S*)-9-ethoxy-1,2,3,4,4a,10b-hexahydro-8-methoxy-2-methylbenzo[c][1,6]naphthyridin-6-yl]-N,N-diisopropylbenzamide which is a mixed PDE3/PDE4 inhibitor which has been prepared and published on by Byk-Gulden, now Altana; arofylline under development by Almirall-Prodesfarma; VM554/UM565 from Vernalis; or T-440 (Tanabe Seiyaku; Fuji, K. et al. J Pharmacol Exp Ther,1998, 284(1): 162), and T2585.

Other possible PDE-4 and mixed PDE3/PDE4 inhibitors include those listed in WO01/13953, the disclosure of which is hereby incorporated by reference.

Suitable anticholinergic agents are those compounds that act as antagonists at the muscarinic receptor, in particular those compounds which are antagonists of the M₁ and M₂ receptors. Exemplary compounds include the alkaloids of the belladonna plants as illustrated by the likes of atropine, scopolamine, homatropine, hyoscyamine; these compounds are normally administered as a salt, being tertiary amines. These drugs, particularly the salt forms, are readily available from a number of commercial sources or can be made or prepared from literature data via, to wit:
Atropine - CAS-51-55-8 or CAS-51-48-1 (anhydrous form), atropine sulfate - CAS-5908-99-6; atropine oxide - CAS-4438-22-6 or its HCl salt - CAS-4574-60-1 and methylatropine nitrate - CAS-52-88-0.
Homatropine - CAS-87-00-3, hydrobromide salt - CAS-51-56-9, methylbromide salt - CAS-80-49-9.
Hyoscyamine (*d*, /) - CAS-101-31-5, hydrobromide salt - CAS-306-03-6 and sulfate salt - CAS-6835-16-1.
Scopolamine - CAS-51-34-3, hydrobromide salt - CAS-6533-68-2, methylbromide salt-CAS-155-41-9.

Preferred anticholinergics include ipratropium (e.g. as the bromide), sold under the name Atrovent, oxitropium (e.g. as the bromide) and tiotropium (e.g. as the bromide) (CAS-139404-48-1). Also of interest are: methantheline (CAS-53-46-3), propantheline bromide (CAS- 50-34-9), anisotropine methyl bromide or Valpin 50 (CAS- 80-50-2), clidinium bromide (Quarzan, CAS-3485-62-9), copyrrolate (Robinul), isopropamide iodide (CAS-71-81-8), mepenzolate bromide (U.S. patent 2,918,408), tridihexethyl chloride (Pathilone, CAS-4310-35-4), and hexocyclium methylsulfate (Tral, CAS-115-63-9). See also cyclopentolate hydrochloride (CAS-5870-29-1), tropicamide (CAS-1508-75-4), trihexyphenidyl hydrochloride (CAS-144-11-6), pirenzepine (CAS-29868-97-1), telenzepine (CAS-80880-90-9), AF-DX 116, or methoctramine, and the compounds disclosed in WO01/04118, the disclosure of which is hereby incorporated by reference.

Suitable antihistamines (also referred to as H₁-receptor antagonists) include any one or more of the numerous antagonists known which inhibit H₁-receptors, and are safe for human use. All are reversible, competitive inhibitors of the interaction of histamine with H₁-receptors. The majority of these inhibitors, mostly first generation antagonists, have a core structure, which can be represented by the following formula:

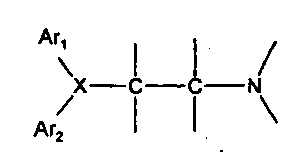

This generalized structure represents three types of antihistamines generally available: ethanolamines, ethylenediamines, and alkylamines. In addition, other first generation antihistamines include those which can be characterized as based on piperizine and phenothiazines. Second generation antagonists, which are non-sedating, have a similar structure-activity relationship in that they retain the core ethylene group (the alkylamines) or mimic the tertiary amine group with piperizine or piperidine. Exemplary antagonists are as follows:
Ethanolamines: carbinoxamine maleate, clemastine fumarate, diphenylhydramine hydrochloride, and dimenhydrinate.
Ethylenediamines: pyrilamine amleate, tripelennamine HCl, and tripelennamine citrate.
Alkylamines: chloropheniramine and its salts such as the maleate salt, and acrivastine.
Piperazines: hydroxyzine HCl, hydroxyzine pamoate, cyclizine HCl, cyclizine lactate, meclizine HCl, and cetirizine HCl.
Piperidines: Astemizole, levocabastine HCl, loratadine or its descarboethoxy analogue, and terfenadine and fexofenadine hydrochloride or another pharmaceutically acceptable salt.
Azelastine hydrochloride is yet another H₁ receptor antagonist which may be used in combination with a PDE4 inhibitor.
Examples of preferred anti-histamines include methapyrilene and loratadine.

The invention thus provides, in a further aspect, a combination comprising a compound of formula (I) a pharmaceutically acceptable salt or solvate thereof together with a PDE4 inhibitor.

The invention thus provides, in a further aspect, a combination comprising a compound of formula (I) a pharmaceutically acceptable salt or solvate thereof together with a corticosteroid.

The invention thus provides, in a further aspect, a combination comprising a compound of formula (I) a pharmaceutically acceptable salt or solvate thereof together with an anticholinergic.

The invention thus provides, in a further aspect, a combination comprising a compound of formula (I) a pharmaceutically acceptable salt or solvate thereof together with an antihistamine.

The invention thus provides, in a further aspect, a combination comprising a compound of formula (I) a pharmaceutically acceptable salt or solvate thereof together with a PDE4 inhibitor and a corticosteroid.

The invention thus provides, in a further aspect, a combination comprising a compound of formula (I) a pharmaceutically acceptable salt or solvate thereof together with an anticholinergic and a PDE-4 inhibitor.

The combinations referred to above may conveniently be presented for use in the form of a pharmaceutical formulation and thus pharmaceutical formulations comprising a combination as defined above together with a physiologically acceptable diluent or carrier represent a further aspect of the invention.

The individual compounds of such combinations may be administered either sequentially or simultaneously in separate or combined pharmaceutical formulations. Appropriate doses of known therapeutic agents will be readily appreciated by those skilled in the art.

According to a further aspect of the invention, there is provided a process for preparing a compound of formula (I), or a salt or solvate thereof which comprises a process (a), (b), (c) or (d) as defined below followed by the following steps in any order:
(i) optional removal of any protecting groups;
(ii) optional separation of an enantiomer from a mixture of enantiomers;
(iii) optional conversion of the product to a corresponding salt, solvate,
(iv) optional conversion of a group R¹, R² and/or R³ to another group R¹, R² and/or R³.

In the following description of synthetic routes, R¹, R², R³, R⁴. R⁵, Z, m and p are as defined for formula (I) and R¹¹, R¹², R¹³ and R¹⁴ are as defined for formula (II) below unless indicated otherwise.

In one general process (a), a compound of formula (I) may be obtained by deprotection of a protected intermediate, for example of formula (II): 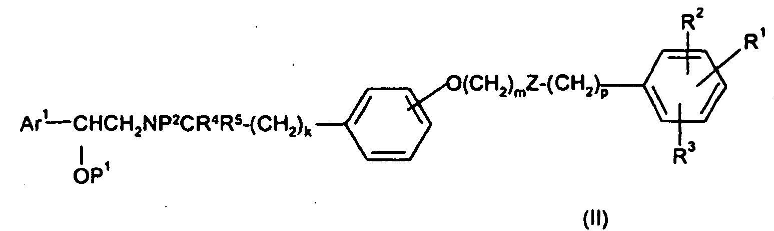 or a salt or solvate thereof, wherein Ar¹, R¹, R², R³, R⁴, R⁵, Z, k, m, and p are as defined for the compounds of formula (I), and P¹ and P² are each independently either hydrogen or a protecting group provided that at least one of P¹ and P² is a protecting group.

Optionally protected forms Ar^{1a} of the preferred groups Ar¹ may be selected from: 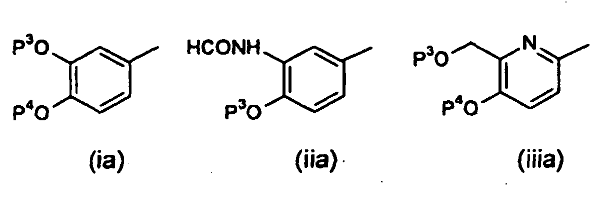 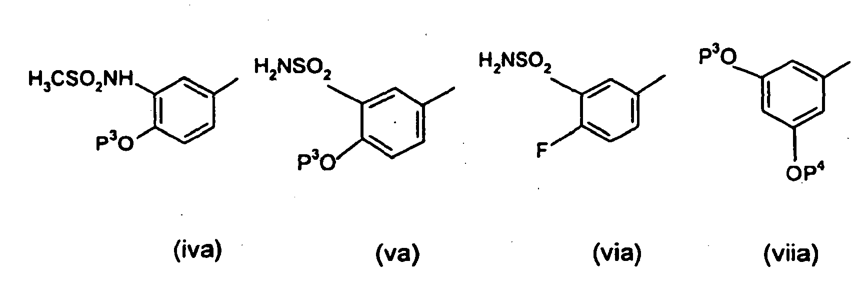 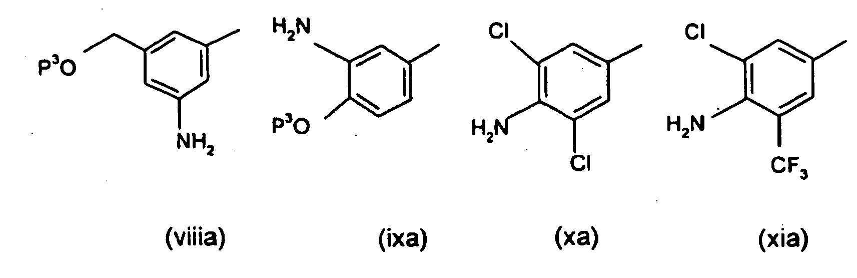 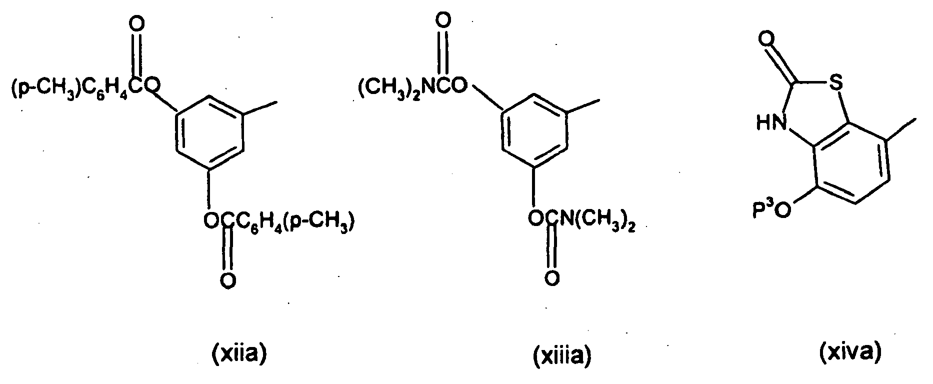 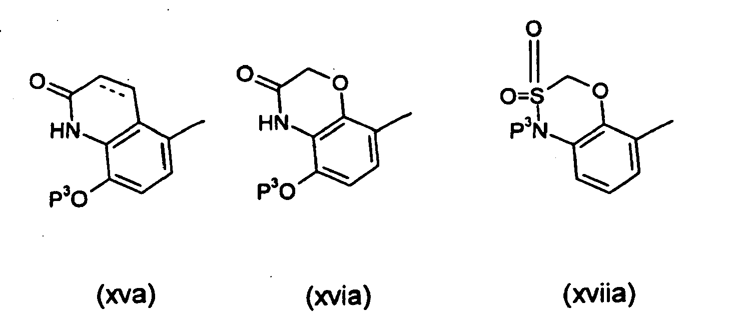 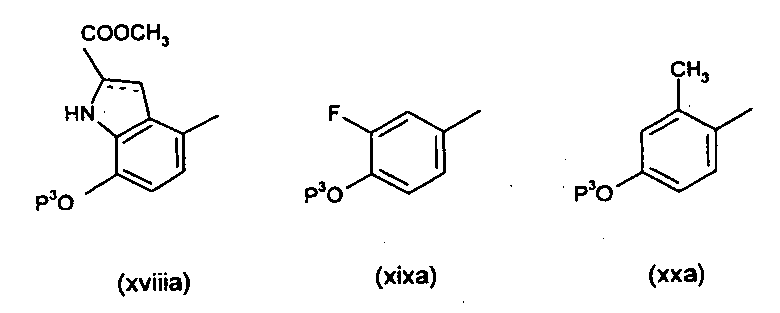 wherein P³ and P⁴ are each independently either hydrogen or a protecting group provided that at least one of P³ and P⁴ is a protecting group, and the dotted line in (xva) and (xviiia) denotes an optional double bond. It will be appreciated that when Ar¹ represents a group (vi), (x), (xi), (xii) or (xiii) no protection is required for Ar¹.

Suitable protecting groups may be any conventional protecting group such as those described in "Protective Groups in Organic Synthesis" by Theodora W Greene and Peter G M Wuts, 3rd edition (John Wiley & Sons, 1999). Examples of suitable hydroxyl protecting groups represented by P³ and P⁴ are esters such as acetate ester, aralkyl groups such as benzyl, diphenylmethyl, or triphenylmethyl, and tetrahydropyranyl. Examples of suitable amino protecting groups represented by P² include benzyl, α-methylbenryl, diphenylmethyl, triphenylmethyl, benzyloxycarbonyl, tert-butoxycarbonyl, and acyl groups such as trichloroacetyl or trifluoroacetyl.

As will be appreciated by the person skilled in the art, use of such protecting groups may include orthogonal protection of groups in the compounds of formula (II) to facilitate the selective removal of one group in the presence of another, thus enabling selective functionalisation of a single amino or hydroxyl function. For example, the -CH(OH) group may be orthogonally protected as - CH(OP¹) using, for example, a trialkylsilyl group such as triethylsilyl. A person skilled in the art will also appreciate other orthogonal protection strategies, available by conventional means as described in Theodora W Greene and Peter G M Wuts (see above).

The deprotection to yield a compound of formula (I), may be effected using conventional techniques. Thus, for example, when P² is an aralkyl group, this may be cleaved by hydrogenolysis in the presence of a metal catalyst (e.g. palladium on charcoal).

When P³ and/or P⁴ is tetrahydropyranyl this may be cleaved by hydrolysis under acidic conditions. Acyl groups represented by P² may be removed by hydrolysis, for example with a base such as sodium hydroxide, or a group such as trichloroethoxycarbonyl may be removed by reduction with, for example, zinc and acetic acid. Other deprotection methods may be found in Theodora W Greene and Peter G M Wuts (see above). In a particular embodiment of process (a) when Ar^{1a} represents a group (iiia), P³ and P⁴ may together represent a protecting group as in the compound of formula (III): 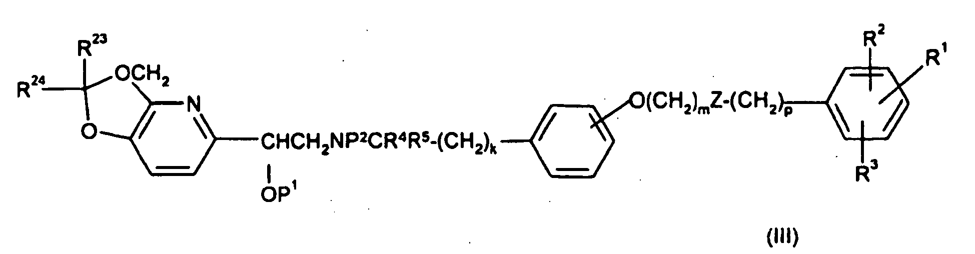 or a salt or solvate thereof, wherein R¹, R², R³, R⁴, R⁶, Z, P¹, P², k, m, and p are as defined for the compound of formula (II), and R²³ and R²⁴ are independently selected from hydrogen, C₁₋₆alkyl, or aryl or R²³ and R²⁴ together form a carbocyclic ring e.g. containing from 5 to 7 carbon atoms. In a preferred aspect, both R²³ and R²⁴ are methyl, or one of R²³ and R²⁴ is hydrogen and the other is phenyl.

The compound of formula (III) may be converted to a compound of formula (I), by hydrolysis with dilute aqueous acid, for example acetic acid or hydrochloric acid in a suitable solvent or by transketalisation in an alcohol, for example ethanol, in the presence of a catalyst such as an acid (for example, toluenesulphonic acid) or a salt (such as pyridinium tosylate) at normal or elevated temperature.

It will be appreciated that the protecting groups P¹, P², P³ and P⁴ (including the cyclised protecting group formed by R²³ and R²⁴ as depicted in formula (III) may be removed in a single step or sequentially. The precise order in which protecting groups are removed will in part depend upon the nature of said groups and will be readily apparent to the skilled worker. Preferably, when R²³ and R²⁴ together form a protecting group as in formula (III) this protecting group is removed together with any protecting group on the CH(OH) moiety, followed by removal of P². However, preferably the nitrogen protecting group is removed first if deprotection is effected using base catalysis.

Certain compounds of formulae (II) and (III) wherein P² is hydrogen may be prepared from the corresponding compound of formula (IV): 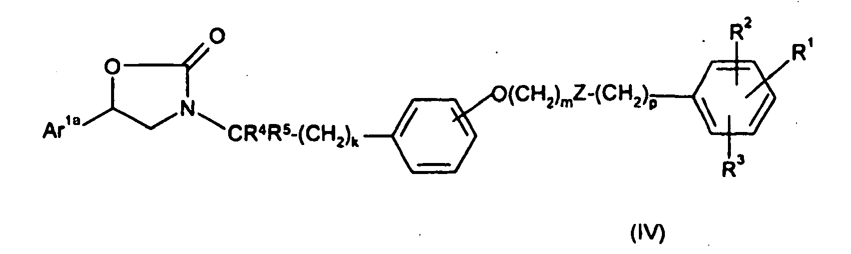 or a salt or solvate thereof, wherein Ar^{1a}, R¹, R², R³, R⁴, R⁵, Z, k, m, and p are as hereinbefore defined.

Other compounds of formula (II) may be prepared by analogous processes.

The conversion of a compound of formula (IV) to a compound of formula (II), (IIa) or (III) may be effected by treatment with a base, for example a non-aqueous base, such as potassium trimethylsilanolate, or an aqueous base such as aqueous sodium hydroxide, in a suitable solvent such as tetrahydrofuran.

A compound of formula (IV) may be prepared by reacting a compound of formula (V): 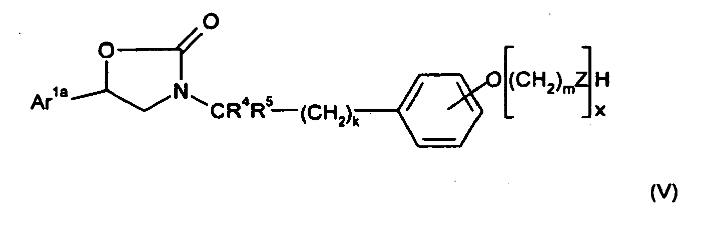 wherein Ar^{1a}, R⁴, R⁵, Z, k and m are as defined hereinbefore for compounds of formula (II);
with a compound of formula (VI): 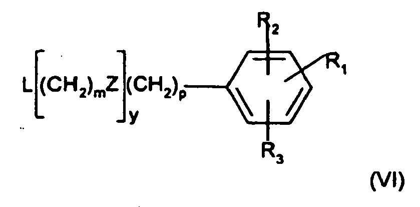 wherein L is a leaving group such as halo (typically chloro, bromo or iodo) or a sulfonate eg. alkylsulfonate (typically methanesulfonate), and x and y each represent 1 or zero such that the sum of x and y is 1. When x is 1, Z preferably represents O.

The reaction of formula (V) and formula (VI) is advantageously effected in the presence of a base such as sodium hydride or a inorganic carbonate, for example, Cs₂CO₃ or K₂CO₃.

Compounds of formula (VI) are commercially available or may be prepared by methods well known to a person skilled in the art.

A compound of formula (V) may be prepared by coupling a compound of formula (VII): 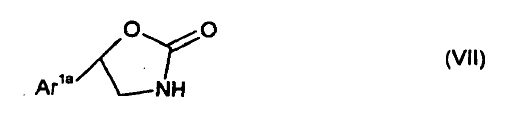 or a salt or solvate thereof, wherein Ar^{1a} is as defined for the compound of formula (II) with a compound of formula (VIII): 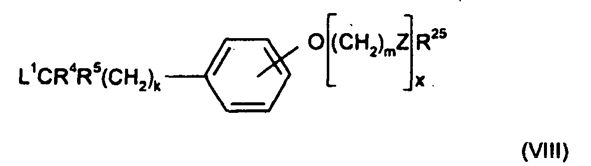 wherein x is zero or 1, L¹ is a leaving group, for example a halo group, (typically bromo or iodo) or a sulfonate such as an alkyl sulfonate (typically methanesulfonate) an aryl sulphonate (typically toluenesulfonate) or a haloalkylsulfonate (typically trifluoromethane sulfonate), and R²⁵ is a hydroxyl protecting group, such as an acyl group. The group R²⁵ may be removed by standard methods; alternatively, the R²⁵ protected compound corresponding to formula (V) may be utilised directly in the reaction with formula (VI).

The coupling of a compound of formula (VII) with a compound of formula (VIII) may be effected in the presence of a base, such as a metal hydride, for example sodium hydride, or an inorganic base such as cesium carbonate, in an aprotic solvent, for example N,N-dimethylformamide. The protecting group R²⁵ may be removed using standard methods, using eg. potassium trimethylsilanolate or sodium hydroxide. Those skilled in the art will appreciate that when potassium silanolate is employed then it is preferable to use only 1 equivalent and mild reaction conditions (room temperature) as an excess of this reagent and high temperature will result in cleavage of the oxazolidinone ring.

A compound of formula (VII) may be prepared for example by the method described in WO02/066422.

A compound of formula (VIII) may be prepared from a compound of formula (IX): 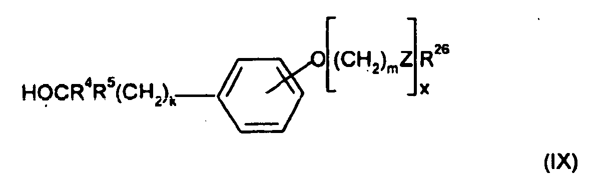 wherein x is zero or 1 and R²⁶ is a hydroxyl protecting group such as aralkyl, typically benzyl, by conventional chemistry, for example by conversion of the hydroxyl group to a mesylate which may itself be converted to bromo by addition of a salt such as tetraalkylammonium bromide in a solvent such as acetonitrile, followed by removal of the protecting group R²⁶ using standard conditions eg. hydrogenation in the presence of palladium on charcoal, and then introduction of R²⁵, for example by reaction with an acyl anhydride.

Compounds of formula (IX) wherein x is zero are known in the art or can readily be prepared by the skilled person using standard methods.

A compound of formula (IX) wherein x is 1 may be prepared from a corresponding compound wherein x is zero by reaction with an appropriate alkylating agent.

Compounds of formulae (II) or (III) may also be prepared according to the general methods described below.

In a further process (b) a compound of formula (I), may be obtained by alkylation of an amine of formula (X): 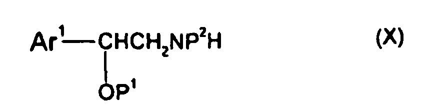 wherein P¹, P² and Ar¹ are each independently either hydrogen or a protecting group, for example as described hereinabove for compounds of formula (II), (IIa) and (III);
with a compound of formula (XI): 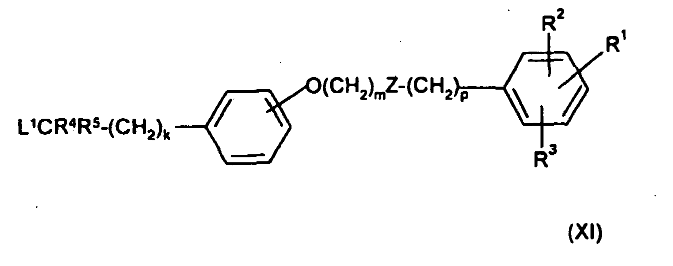 wherein R¹, R², R³. R⁴, R⁵, Z, k, m, and p are as defined for compounds of formula II and L¹ is a leaving group as herein before defined for the compound of formula (VIII); followed by removal of any protecting groups present by conventional methods as described above for the deprotection of compounds of formula (II), (IIa) and (III). For speed of reaction, L¹ is preferably bromo or is converted to bromo in situ, from the corresponding compound wherein L¹ is methanesulphonate, for example by addition of tetrabutylammonium bromide to the reaction mixture. In this process P² is preferably hydrogen.

A compound of formula (I), may be formed directly (when in the compound of formula (X) P¹, P² and where appropriate P³ and P⁴ are each hydrogen) or via a compound of formula (II), (IIa) or (III) which may or may not be isolated (when in the compound of formula (X) at least one of P¹, P², P³ and P⁴ is a protecting group).

The reaction of compounds of formulae (X) and (XI) is optionally effected in the presence of an organic base such as a trialkylamine, for example, diisopropylethylamine, and in a suitable solvent for example N,N-dimethylformamide, or acetonitrile.

Compounds of formula (X) are known in the art (for example EP-A 0947498) or may be readily prepared by a person skilled in the art, using known methods for example as described in WO02/066422.

Further details concerning preparation of compounds (X) wherein Ar¹ is a group (iv) can be found in DE3524990; concerning the preparation of compounds (X) wherein Ar¹ is a group (i), (vii), and (xv) in EP-A-162576; concerning the preparation of compounds (X) wherein Ar¹ is a group (iii) in EP-A-220054; concerning the preparation of compounds (X) wherein Ar¹ is a group (x) in GB2165542 and concerning the preparation of compounds (X) wherein Ar¹ is a group (c) in GB2230523.

Compounds of formula (XI) may be prepared by general methods described hereinabove, as will be evident to a person skilled in the art, for example using methods similar to those used in the preparation of compounds (IX) and the reaction of compounds (V) and (VI).

In a further process (c) a compound of formula (I), may be prepared by reacting a compound of formula (XII): 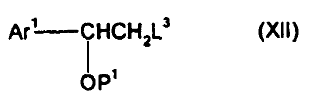 wherein P¹ and Ar¹ are as hereinbefore defined and L³ is a leaving group, with an amine of formula (XIII): 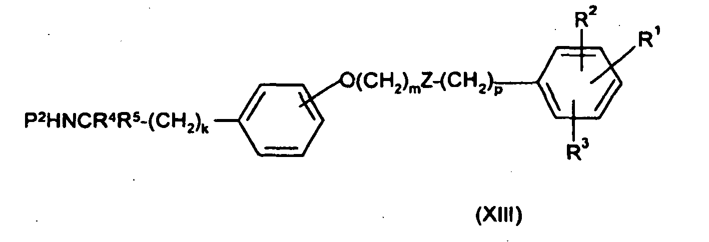 wherein R¹, R², R³, R⁴, R⁵, Z, k, m, p and P² are as hereinbefore defined;
followed by removal of any protecting groups present by conventional methods as described above for the deprotection of compounds of formula (II) and (IIa).

The reaction may be effected using conventional conditions for such displacement reactions.

Compounds of formula (XII) may be prepared by methods known in the art.

Compounds of formula (XIII) may be prepared by reacting a compound of formula (XI) with an amine P²NH₂.

According to a further process (d) compounds of formula (I) wherein one of R⁴ and R⁵ represents alkyl may be prepared by reacting a compound of formula (X): 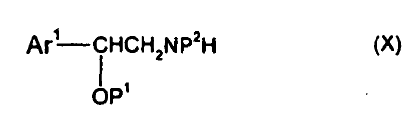 as hereinbefore defined,
with a compound of formula (XIV): 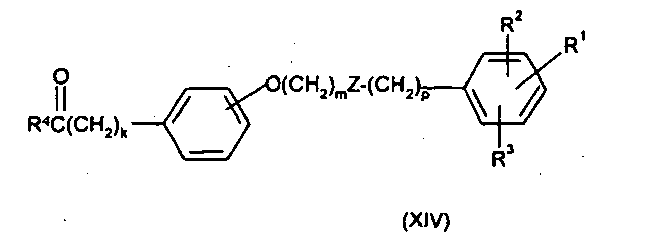 under conditions suitable to effect reductive amination, for example in the presence of a reducing agent such as a borohydride, typically tetramethylammonium (triacetoxy) borohydride.

A compound of formula (XIV) may be prepared by alkylation of a compound of formula (XV) 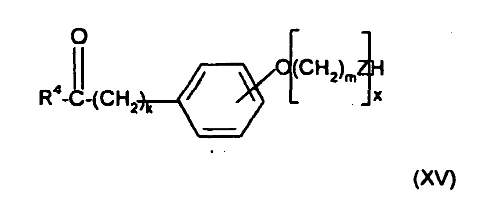 wherein x is zero or 1, with a compound of formula (VI) as hereinbefore defined using methods analogous to those described hereinbefore for the preparation of compounds of formula (IV). In this reaction protection of the carbonyl may be necessary to effect efficient alkylation at the desired position.

Compounds of formula (XV) wherein x is zero are commercially available or may readily be prepared by conventional methods. Compounds of formula (XV) where x is 1 may be prepared from a corresponding compound wherein x is zero by appropriate alkylation.

It will be appreciated that at any convenient stage in the preparation of a compound of formula (I) one or more of the substituents R¹, R² and R³ may, if appropriate, be converted into a different substituent. Conveniently such conversion may be effected on a compound of formula (IV) prior to the deprotection stages.

Thus for example a compound wherein R¹ represents -NH₂ may be converted into a compound wherein R¹ represents XN R⁶C(O)N R⁷ R⁸ by reaction with an appropriate isocyanate or into a compound wherein R¹ represents L-XN R⁶(CO)N(CO)N R⁷ R⁸ using excess isocyanate - similarly, amide and sulfonamide derivatives may be formed by reaction with an appropriate acyl or sulfonyl chloride or anhydride. Alternatively a simple amide substituent may be prepared from the corresponding nitrile, by treatment with a base such as potassium trimethylsilanolate. Other transformations will be apparent to those skilled in the art, and may be effected by conventional reactions.

It will be appreciated that in any of the routes (a) to (d) described above, the precise order of the synthetic steps by which the various groups and moieties are introduced into the molecule may be varied. It will be within the skill of the practitioner in the art to ensure that groups or moieties introduced at one stage of the process will not be affected by subsequent transformations and reactions, and to select the order of synthetic steps accordingly.

The enantiomeric compounds of the invention may be obtained (i) by separation of the components of the corresponding racemic mixture, for example, by means of a chiral chromatography column, enzymic resolution methods, or preparing and separating suitable diastereoisomers, or (ii) by direct synthesis from the appropriate chiral intermediates by the methods described above.

Optional conversions of a compound of formula (I), to a corresponding salt may conveniently be effected by reaction with the appropriate acid or base. Optional conversion of a compound of formula (I), to a corresponding solvate may be effected by methods known to those skilled in the art.

According to a further aspect, the present invention provides novel intermediates for the preparation of compounds of formula (I), for example, compounds of formula (IV).

For a better understanding of the invention, the following Examples are given by way of illustration.

### SYNTHETIC EXAMPLES

Throughout the examples, the following abbreviations are used:
LCMS: Liquid Chromatography Mass Spectrometry
HPLC: High Performance Liquid Chromatography
RT: retention time
DCM: dichloromethane
EtOAc: ethyl acetate
EtOH: ethanol
DMAP: N,N-Dimethylaminopyridine
DMF: N,N-Dimethylformamide
DIPEA: diisopropylethylamine
AcOH: acetic acid
PPh₃: triphenylphosphine
MeOH: methanol
THF: tetrahydrofuran
h: hour(s)
min: minute(s)

All temperatures are given in degrees centigrade.
Flash silica gel refers to Merck Art No. 9385; silica gel refers to Merck Art No. 7734
Biotage refers to prepacked silica gel cartridges containing KP-Sit run on flash 12i chromatography module.
Solid Phase Extraction (SPE) columns are pre-packed cartridges used in parallel purifications, normally under vacuum. These are commercially available from Varian.
SCX cartridges are Ion Exchange SPE columns where the stationary phase is polymeric benzene sulfonic acid. These are used to isolate amines.
LCMS was conducted on a Supelcosil LCABZ+PLUS column (3.3cm x 4.6mm ID) eluting with 0.1% HCO₂H and 0.01 M ammonium acetate in water (solvent A) and 0.05% HCO₂H 5% water in acetonitrile (solvent B), using the following elution gradient 0.0-7 min 0%B, 0.7-4.2 min 100%B, 4.2-5.3 min 100%B, 5.3-5.5min 0%B at a flow rate of 3mUmin. The mass spectra were recorded on a Fisons VG Platform spectrometer using electrospray positive and negative mode (ES+ve and ES-ve).

Preparative mass directed HPLC was conducted on a Waters FractionLynx system comprising of a Waters 600 pump with extended pump heads, Waters 2700 autosampler, Waters 996 diode array and Gilson 202 fraction collector on a 10 cm X 2.54 cm ID ABZ+ column, eluting with 0.1% formic acid in water (solvent A) and 0.1% formic acid in acetonitrile (solvent B), using the following elution gradient: 0.0-1.0 min 15%B, 1.0-10.0 min 55%B, 10.0-14.5 min 99%B, 14.5-14.9 min 99%B, 14.9-15.0 min 15%B at a flow rate of 20 ml/min and detecting at 200-320 nm at room temperature. Mass spectra were recorded on Micromass ZMD mass spectrometer using electrospray positive and negative mode, alternate scans. The software used was *MassLynx 3.5* with *OpenLynx* and *FractionLynx* options.

### Example 1

### 3-{[2-(4-{2-[((2R)-2-Hydroxy-2-{4-hydroxy-3-[(methylsulfonyl)amino]phenyl}ethyl)amino]ethyl}phenoxy)ethoxy]methyl}benzamide

### i) N-12-Hydroxy-5-((1R)-1-hydrxy-2-4[(1S)-2-hydroxy-1-phenylethyl]amino}ethyl]phenyl]methanesulfonamide

A solution of *N*-[5-(bromoacetyl)-2-hydroxyphenyl]methanesulfonamide *(*J. Med. Chem. 1967, 10, 462-72) (1.15g) in dry DMF (30mL) was treated with DIPEA (1.06mL) and (*S*)-phenylglycinol (474mg) and the reaction mixture stirred at room temperature for 4h. The reaction mixture was concentrated *in vacuo* and the residue resuspended in methanol (50mL). The reaction mixture was cooled to 0°C and treated with CaCl₂ (1.27g). The reaction mixture was stirred at 0°C for 30 min prior to portionwise addition of NaBH₄ (218mg) ensuring that the temperature did not rise above 10°C. After complete addition the reaction mixture was allowed to warm to room temperature and stirred for a further 74h. The reaction mixture was concentrated *in vacuo* and the residue partitioned between EtOAc and water. The organic phase was dried and concentrated *in vacuo.* The mixture was purified by chromatography (SPE, gradient from DCM to DCM-MeOH-NH₃(aq) 100:10:1) afforded *the title compound* (85mg). LCMS RT= 2.48min

### ii) N-{5-[(1R)-2-Amino-1-hydroxyethyl]-2-hydroxyphenyl}methanesulfonamide

Palladium hydroxide (40mg, 50% water) was flushed with nitrogen and treated with a solution of *N*-[2-hydroxy-5-((1*R*)-1-hydroxy-2-{[(1*S*)-2-hydroxy-1-phenylethyl]amino}ethyl)phenyl]methanesulfonamide (400mg) in methanol (80mL) and acetic acid (0.5mL). The reaction mixture was stirred under hydrogen for 16 h prior to flushing the reaction mixture with nitrogen and filtering to remove the catalyst and concentrating *in vacuo*. The residue was purified by chromatography (OASIS, eluted with water, 5% MeOH in water 50% MeOH in water and MeOH) to give *the title compound* (182mg). δ_{H} (400MHz, CD₃OD) 7.38 (1H, d, *J* 2Hz), 7.12 (1 H, dd, *J* 2, 8Hz), 6.90
(1H, d, *J* 8Hz), 4.78 (1H. dd, J 2, 10Hz), 3.08 (1H, dd, J 2, 15Hz), 2.98 (1H, bd, J 10Hz), 2.93 (3H, s).

### iii) 3-((2-Hydroxyethoxylmethyllbenzonitrile

Ethylene glycol (6.2g) was treated with sodium hydride (60% dispersion in oil, 480mg) and stirred for 30 min. 3-(Bromomethyl)benzonitrile (1.96g) was added and the reaction mixture heated at 80°C for 15 h. The reaction mixture was cooled to room temperature and quenched with water. The resultant mixture was partitioned between water and ether. The aqueous phase was extracted with ether and the combined organic phase dried and concentrated *in vacuo.* The residue was purified by chromatography (SPE, eluted with gradient between cyclohexane and 50% EtOAc in cyclohexane) to give *the title compound* (780mg). LCMS RT= 2.22 min.

### iv) 3-[(2-Bromoethoxy)methyl]benzonitrile

A solution of 3-[(2-hydroxyethoxy)methyl]benzonftrile (500mg) in dry DCM (20mL) was cooled to 0°C and treated with PPh₃ (1.86g) and CBr₄ (2.53g). The reaction mixture was stirred at 0°C for 30 min and room temperature for 30min prior to concentration *in vacuo.* The residue was purified by chromatography (SPE, eluted with a gradient between cyclohexane and 50% Et₂O in cyclohexane) to give *the title compound* (639mg). LCMS RT= 3.0 min.

### v) 3-({2-[4-(2-Hydroxyethyl)phenoxy]ethoxy}methyl)benzonitrile

A solution of 4-(2-hydroxyethyl)phenol (414mg) in dry DMF (5mL) was treated with cesium carbonate (815mg) and 3-[(2-bromoethoxy)methyl]benzonitrile (600mg) and heated at 150°C in a microwave (CEM explorer @ 150 watts) for 10 min. The reaction mixture was poured into water and extracted into EtOAc. The organic phase was washed twice with 2N NaOH_{(aq)} dried and concentrated *in vacuo*. The residue was purified by chromatography (SPE, eluted with a gradient between cyclohexane and EtOAc) to give *the title compound* (639mg). LCMS RT= 2.92 min.

### vi) 3-({2-[4-(2-Bromoethyl)phenoxy]ethoxy}methyl)benzonitrile

A solution of 3-({2-[4-(2-hydroxyethyl)phenoxy]ethoxy}methyl)benzonitrile (300mg) in dry DCM (10mL) was cooled to 0°C and treated with PPh₃ (663mg) and CBr₄ (840mg). The reaction mixture was stirred at 0°C for 30 min and room temperature for 16h prior to concentration *in vacuo.* The residue was purified by chromatography (SPE, eluted with a gradient between cyclohexane and 50% Et₂O in cyclohexane) to give *the title compound* (424mg). LCMS RT= 3.59 min.

### vii) N-[5-((1R)-2-{[2-(4-(2-[(3-Cyanobenzyl)oxy]ethoxy}phenyl)ethyl]amino}-1-hydroxyethyl)-2-hydroxyphenyl]methanesulfonamide

A solution of *N*-{5-[(1*R*)-2-amino-1-hydroxyethyl]-2-hydroxyphenyl}methanesulfonamide (49mg) in dry DMF (2mL) was treated with DIPEA (84µL) and 3-({2-[4-(2-bromoethyl)phenoxy]ethoxy}methyl)benzonitrile (87mg). The reaction mixture was heated at 50°C for 48 h. The reaction mixture was concentrated *in vacuo* and purified by mass directed HPLC to afford *the title compound* (24mg). LCMS RT= 2.54 min ES+ve m/z 525 (MH)⁺

### viii) 3-{[2-(4-{2-[((2R)-2-Hydroxy-2-(4-hydroxy-3-[(methylsulfonyl)amino]phenyl}ethyl)aminolethyl}phenoxy)ethoxy]methyl}benzamide

A solution of *N*-[5-((1*R*)-2-{[2-(4-{2-[(3-cyanobenzyl)oxy]ethoxy}phenyl)ethyl]amino}-1-hydroxyethyl)-2-hydroxyphenyl]methanesulfonamide (24mg) in dry THF (1mL) was treated with potassium trimethylsilanolate (34mg) and heated at 150°C in a microwave (CEM explorer @ 150 watts) for 2 min. The reaction mixture was concentrated *in vacuo* and the residue partitioned between EtOAc and water. The organic phase was dried and concentrated *in vacuo.* The residue was purified by mass directed HPLC to afford *the title compound* (24mg). LCMS RT= 2.16 min ES+ve m/z 543 (MH) ⁺

### Example 2

### N-{2-Hydroxy-5-[(1R)-1-hydroxy-2-({2-[4-(4-phenylbutoxy)phenyl]ethyl}amino)ethyl]phenyl}methanesulfonamide

### i) 2-[4-(4-Phenylbutoxy)phenyl]ethanol

Prepared similarly to Example 1 v) from (4-bromobutyl)benzene
LCMS RT= 3.51 min.

### ii) 1-(2-Bromoethyl)-4-(4-phenylbutoxy)benzene

Prepared similarly to Example 1 vi), LCMS RT= 4.06 min

### iii) N-{2-Hydroxy-5-[(1R)-1-hydroxy-2-({2-[4-(4-Phenylbutoxy)phenyl]ethyl}amino)ethyl]phenyl}methanesulfonamide

Prepared similarly to Example 1 vii), LCMS RT= 2.81 min ES+ve m/z 498 (MH)⁺

### Example 3

### N-(5-{(1R)-2-[(2-{4-[2-(Benzyloxy)ethoxy]phenyl}ethyl)amino]-1-hydroxyethyl}-2-hydroxyphenyl)methanesulfonamide

### i) 2-{4-[2-(Benzyloxy)ethoxy]phenyl)ethanol

Prepared similarly to Example 1 v) from [(2-bromoethoxy)methyl]benzene, LCMS RT= 3.04 min.

### ii) 1-[2-(Benzyloxy)ethoxy]-4-(2-bromoethyl)benzene

Prepared similarly to Example 1 vi), LCMS RT= 3.73 min

### iii) N-(5-{(1R)-2-[(2-{4-[2-(Benzyloxy)ethoxy]phenyl}ethyl)amino]-1-hydroxyethyl}-2-hydroxyphenyl)methanesulfonamide

Prepared similarly to Example 1 vii), LCMS RT= 2.48 min ES+ve m/z 500 (MH)⁺

### Example 4

### 3-({2-[4-(2-{[(2R)-2-(3-Fluoro-4-hydroxyphenyl)-2-hydroxyethyl]amino}ethyl)phenoxy]ethoxy}methyl)benzamide

### i) 2-Azido-1-[4-(benzyloxy)-3-fluorophenyl]ethanone

A solution of 2-bromo-1-[4-(benzyloxy)-3-fluorophenyl]ethanone (1g) in dry DMF (2.5mL) was cooled to 15°C and treated portionwise with sodium azide (220mg). After complete addition the reaction mixture was stirred for a further 1h. The reaction mixture was partitioned between EtOAc and water. The organic phase was washed with water and the combined aqueous phase back extracted with EtOAc. The combined organic phase was washed with sat. NaHCO₃(aq) three times and the combined washes back extracted with EtOAc. The combined organic phase was washed with brine, dried and concentrated *in vacuo.* The residue was purified by chromatography (silica, eluted with hexane:EtOAc, 4:1 and 2:1) to give *the title compound* (810mg). LCMS RT= 3.61 min.

### ii) (1R)-2-Azido-1-[4-(benzyloxy)-3-fluorophenyl]ethanol

Borane-dimethylsulphide solution in THF (2N, 0.03 mL) was added to a solution of (R)-2-methyl-CBS-oxazaborolidine in toluene (1M, 0.06mL) at 0°C with stirring. The reaction mixture was stirred for 15 min prior to the dropwise addition of a solution of 2-azido-1-[4-(benzyloxy)-3-fluorophenyl]ethanone (100mg) in THF. Further Borane-dimethylsulphide in THF (2N, 0.03mL) was added dropwise and the reaction mixture stirred at 0°C for 2h. 2N HCl(aq) (2mL) was added dropwise and the reaction mixture stirred for 10min prior to partitioning the reaction mixture between ether and water. The organic phase was washed twice with 2N HCI(aq), three times with sat. NaHCO₃(aq), water and brine. The organic phase was dried and concentrated *in vacuo.* The residue was purified by chromatography (silica, eluted with DCM) to give *the title compound* (470mg). LCMS RT= 3.36 min.

### iii) (1R)-2-Amino-1-[4-(benzyloxy)-3-fluorophenyl]ethanol

A solution of (1*R*)-2-azido-1-[4-(benzyloxy)-3-fluorophenyl]ethanol (410mg) in THF (8mL) and water (2mL) was treated with PPh₃ (410mg) and stirred for 1h prior to addition of further with PPh₃ (220mg). After stirring for a further 4h the reaction mixture was concentrated in vacuo and the residue partitioned between EtOAc and water. The organic phase was washed three times with 5% NaHCO₃(aq) dried and concentrated *in vacuo.* The residue was purified by chromatography (silica, washed with DCM and eluted with 1% MeOH in DCM, 2% MeOH in DCM, 5% MeOH + 0.5% Et₃N in DCM, & 20% MeOH + 1% Et₃N in DCM) to give *the title compound* (260mg). LCMS RT= 2.16 min.

### iv) 4-[(1R)-2-Amino-1-hydroxyethyl]-2-fluorophenol

Palladium on carbon (10% Pd by weight, wet, 50mg) was flushed with nitrogen and treated with a solution of (1*R*)-2-amino-1-[4-(benzyloxy)-3-fluorophenyl]ethanol (500mg) in ethanol (25mL), EtOAc (25mL) and acetic acid (10mL). The reaction mixture was stirred under hydrogen for 5 h prior to flushing the reaction mixture with nitrogen and filtering to remove the catalyst and concentrating *in vacuo.* The residue was purified by chromatography (SCX, eluted with DCM, MeOH and DCM-MeOH-NH₃(aq) 100:10:1) to give *the title compound* (308mg). δ_{H} (400MHz, CD₃OD) 7.05 (1 H, dd, *J* 2, 12Hz), 6.94 (1H, dd, *J* 2, 9Hz), 6.86 (1H, t, *J* 9Hz), 4.54 (1H, dd, J 5, 8Hz), 2.78 (1H, d, J 5Hz), 2.77 (1H, d, J 8Hz).

### v) 3-({2-[4-(2-{[(2R)-2-(3-Fluoro-4-hydroxyphenyl)-2-hydroxyethyl]amino}ethyl)phenoxy]ethoxy}methyl)benzonitrile

Similarly prepared to Example 1 vii, LCMS RT= 2.59 min.

### vi) 3-({2-[4-(2-{[(2R)-2-(3-Fluoro-4-hydroxyphenyl)-2-hydroxyethyl]amino}ethyl)phenoxy]ethoxy}methyl)benzamide

Similarly prepared to Example 1 viii, LCMS RT= 2.21 min ES+ve m/z 468 (MH)⁺

### Example 5

### 4-{(1R)-2-[(2-{4-[2-(Benzyloxy)ethoxy]phenyl}ethyl)amino]-1-hydroxyethyl)-2-fluorophenol

Similarly prepared to Example 1vii) from the compound of Example 4iv), LCMS RT= 2.65 min ES+ve m/z 425 (MH)⁺

### Example 6

### 2-Fluoro-4-[(1R)-1-hydroxy-2-({2-[4-(4-phenylbutoxy)phenyl]ethyl}amino)ethyl]phenol

### i) (1R)-1-[4-(Benzyloxy)-3-fluorophenyl]-2-({2-[4-(4-phenylbutoxy)phenyl]ethyl}amino)ethanol

Prepared similarly to Example 2 using intermediate 4 iii) LCMS RT= 3.25 min.

### ii) 2-Fluoro-4-[(1R)-1-hydroxy-2-({2-[4-(4-phenylbutoxy)phenyl]ethyl}amino)ethyl]phenol

Palladium on carbon (10% Pd by weight, wet, 20mg) was flushed with nitrogen and treated with a solution of (1*R*)-1-[4-(benzyloxy)-3-fluorophenyl]-2-({2-[4-(4-phenylbutoxy)phenyl]ethyl}amino)ethanol (18mg) in ethanol (10mL) and acetic acid (1mL). The reaction mixture was stirred under hydrogen for 5 h prior to flushing the reaction mixture with nitrogen and filtering to remove the catalyst and concentrating *in vacuo.* The residue was purified by chromatography (SCX, eluted with DCM, MeOH and DCM-MeOH-NH₃(aq) 100:10:1) to give *the title compound* (13.5mg). LCMS RT= 2.85 min ES+ve m/z 423 (MH)⁺

### Example 7

### 3-[(2-{4-(2-({2-Hydroxy-2-(5-hydroxy-6-(hydroxymethyl)pyridin-2-yl]ethyl}amino)ethyl]phenoxy}ethoxy)methyl]benzamide

### i) 3-({2-[4-(2-{[2-Hydroxy-2-(2-phenyl-4H-[1,3]dioxino[5,4-b]pyridin-6-yl)ethyl]amino}ethyl)phenoxy]ethoxy}methyl)benzamide

Prepared similarly to Example 1 using 2-amino-1-(2-phenyl-4*H*-[1,3]dioxino[5,4-*b*]pyridin-6-yl)ethanol LCMS RT= 2.56 min

### ii) 3-[(2-(4-[2-({2-Hydroxy-2-[5-hydroxy-6-(hydroxymethyl)pyridin-2-yl]ethyl}amino)ethyl]phenoxy}ethoxy)methyl]benzamide

A solution of 3-({2-[4-(2-([2-hydroxy-2-(2-phenyl-4*H*-[1,3]dioxino[5,4-b]pyridin-6-yl)ethyl]amino}ethyl)phenoxylethoxy)methyl)benzamide (51 mg) in AcOH (2mL) and water (2mL) was heated at 80°C for 30 min prior to cooling and concentration in vacuo. The residue was purified by mass directed HPLC to afford prod (53mg). LCMS RT= 2.07 min ES+ve m/z 481 (MH)⁺

### Example 8

### 6-{2-[(2-{4-[2-(Benzyloxy)ethoxy]phenyl}ethyl)amino]-1-hydroxyethyl}-2-(hydroxymethyl)pryridin-3-ol

### i) 2-({2-[4-(4-Phenylbutoxy)phenyl]ethyl}amino)-1-(2-phenyl-4H-[1,3]dioxino[5,4-b]pyridin-6-yl)ethanol

Prepared similarly to Example 3, LCMS RT= 2.98 min

### ii) 6-{2-[(2-{4-[2-(Benzyloxy)ethoxy]phenyl}ethyl)amino]-1-hydroxyethyl)-2-(hydroxymethyl)pyridin-3-ol

Prepared similarly to Example 7 ii), LCMS RT= 2.35 min ES+ve m/z 438 (MH)⁺

### Example 9

### 2-(Hydroxymethyl)-6-[1-hydroxy-2-({2-[4-(4-phenylbutoxy)phenyl]ethyl}amino)ethyl]pyridin-3-ol

### i) 2-({2-[4-(4-Phenylbutoxy)phenyl]ethyl}amino)-1-(2-phenyl-4H-[1.3]dioxino[5.4-b]pyridin-6-yl)ethanol

Prepared similarly to Example 3, LCMS RT= 3.14 min

### ii) 2-(Hydroxymethyl)-6-[1-hydroxy-2-({2-[4-(4-phenylbutoxy)phenyl]ethyl}amino)ethyl]pyridin-3-ol

Prepared similarly to Example 7 ii, LCMS RT= 2.72 min ES+ve m/z 436 (MH)⁺

### BIOLOGICAL ACTIVITY

### In vitro measurements of compound potency and intrinsic activity at the human Beta 1, 2 and 3 receptors.

### Method 1

The potencies of the compounds were determined using frog melanophores transfected with the human beta 2 adrenoreceptor. The cells were incubated with melatonin to induce pigment aggregation. Pigment dispersal was induced by compounds acting on the human beta 2 adrenoreceptor. The beta 2 agonist activity of test compounds was assessed by their ability to induce a change in light transmittance across a melanophore monolayer (a consequence of pigment dispersal). At the human beta 2 adrenoreceptor, compounds of said examples had IC₅₀ values below 1 µM.

### Method 2

Potency of compounds of the invention at the human beta 2, 1 and 3 receptors was also determined using Chinese hamster ovary cells co-expressing the human receptor with a reporter gene. Studies were performed using either whole cells or membranes derived from those cells.

The three beta-receptors are coupled via the Gs G-protein to cause a stimulation of adenylate cyclase resulting in increased levels of cAMP in the cell. For direct cAMP measurements either membranes or cells have been used with either the HitHunter enzyme fragment complementation kit (DiscoveRx) or the FP² fluorescence polarisation kit (Perkin Elmer) to quantify the levels of cAMP present. These assays provide a measure of agonist potency and intrinsic activity of the compounds at the various receptors.

The reporter gene in the cells has also been used to quantify potency at the beta 1 and 3 receptors. This is a reporter of cAMP levels using the cAMP response element upstream of a firefly luciferase gene. After stimulation of the receptor with an agonist an increase in the level of luciferase is measured as a quantification of the level of cAMP in the cell.

In this assay the potency of compounds at the human beta-2 receptor is expressed as a pEC₅₀ value. Representative compounds had a pEC₅₀ of >6.

## Claims

1. A compound of formula (I) 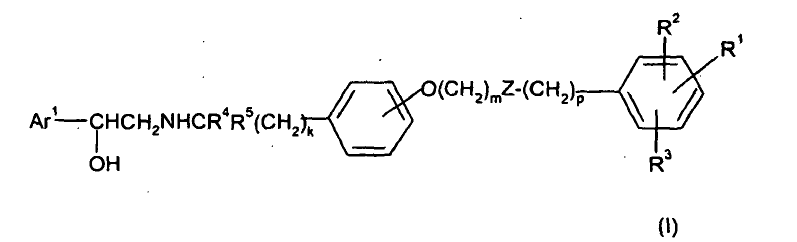 or a salt or solvate thereof, wherein:
k is an integer of from 1 to 3;
m is an integer of from 2 to 4;
p is an integer of from 1 to 4;
Z is O or CH₂-
R¹ is selected from hydrogen, C₁₋₆alkyl, hydroxy, C₁₋₆alkoxy, cyano, nitro, halo, C₁₋₆haloalkyl, XCO₂R⁸, -XC(O)NR⁷R⁸, -XNR⁶C(O)R⁷, -XNR⁶C(O)NR⁷R⁸, -XNR⁶C(O)NC(O)NR⁷R⁸, -XNR⁶SO₂R⁷, -XSO₂NR⁹R¹⁰, XSR⁶, XSOR⁶, XSO₂R⁶, XNR⁶SO₂NR⁷R⁸, XNR⁶SO₂NR⁷COOR⁷,
-XNR⁷R⁸, -XNR⁶C(O)OR⁷,
or R¹ is selected from -X-aryl, -X-hetaryl, or -X-(aryloxy), each optionally substituted by 1 or 2 groups independently selected from hydroxy, C₁₋₆alkoxy, halo, C₁₋₆alkyl, C₁₋₆haloalkyl, -NR⁶C(O)R⁷, SR⁶, SOR⁶, -SO₂R⁶, -SO₂NR⁹R¹⁰, -CO₂R⁸, -N R⁷R⁸, or hetaryl optionally substituted by 1 or 2 groups independently selected from hydroxy, C₁₋₆alkoxy, halo, C₁₋₆alkyl, or C₁₋₆haloalkyl;
X is -(CH₂)_{q}- or C₂₋₆ alkenylene;
q is an integer from 0 to 6;
R⁶and R⁷ are independently selected from hydrogen, C₁₋₆alkyl, C₃₋₇cycloalkyl, aryl, hetaryl, hetaryl(C₁₋₆alkyl)- and aryl(C₁₋₆alkyl)- and R⁶ and R⁷ are each independently optionally substituted by 1 or 2 groups independently selected from halo, C₁₋₆alkyl, C₃₋₇ cycloalkyl, C₁₋₆ alkoxy, C₁₋₆haloalkyl, -NHC(O)(C₁₋₆alkyl), -SO₂(C₁₋₆alkyl), -SO₂(aryl), -CO₂H, -CO₂(C₁₋₄alkyl), -NH₂, -NH(C₁₋₆alkyl), aryl(C₁₋₆alkyl)-, aryl(C₂-₆alkenyl)-, aryl(C₂₋₆alkynyl)-, hetaryl(C₁₋₆alkyl)-, -NHSO₂aryl, -NH(hetarylC₁₋₆alkyl), - NHSO₂hetaryl,
-NHSO₂(C₁₋₆alkyl), -NHC(O)aryl, or -NHC(O)hetaryl:
R⁸ is selected from hydrogen, C₁₋₆alkyl and C₃₋₇ cycloalkyl;
or R⁷ and R⁸, together with the nitrogen atom to which they are bonded, form a 5-, 6-or 7- membered nitrogen - containing ring;
R⁹ and R¹⁰ are independently selected from hydrogen, C₁₋₆alkyl, C₃₋₇cycloalkyl, aryl, hetaryl, hetaryl(C₁₋₆alkyl)- and aryl(C₁₋₆alkyl)-, or R⁹ and R¹⁰, together with the nitrogen to which they are bonded, form a 5-, 6-, or 7- membered nitrogen containing ring;
and R⁹ and R¹⁰ are each optionally substituted by one or two groups independently selected from halo, C₁₋₆alkyl, C₃₋₇cycloalkyl and C₁₋₆haloalkyl;
R² is selected from hydrogen, hydroxy, C₁₋₆alkyl, C₁₋₆alkoxy, halo, aryl, aryl(C₁₋₆alkyl)-, C₁₋₆haloalkoxy, and C₁₋₆haloalkyl;
R³ is selected from hydrogen, hydroxy, C₁₋₆alkyl, C₁₋₆alkoxy, halo, aryl, aryl(C₁₋₆alkyl)-, C₁₋₆haloalkoxy, and C₁₋₆haloalkyl;
R⁴ and R⁵ are independently selected from hydrogen and C₁₋₄ alkyl with the proviso that the total number of carbon atoms in R⁴ and R⁵ is not more than 4: and
Ar¹ is a group selected from 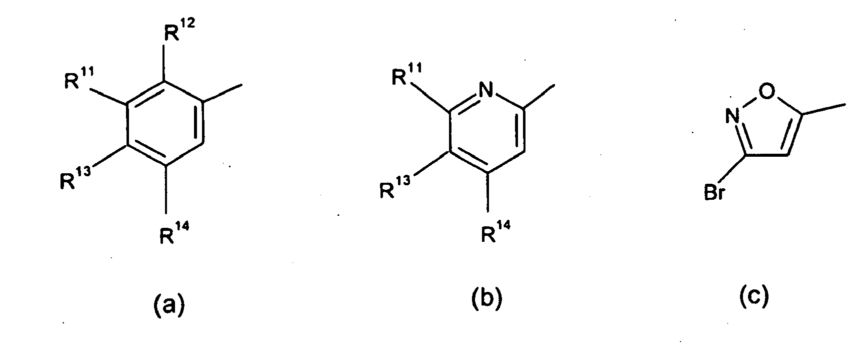
and 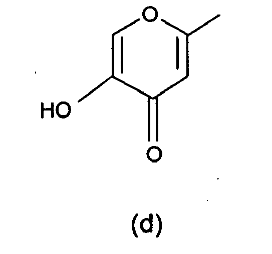 wherein R¹¹ represents halogen, -(CH₂)ₙOR¹⁵, -NR¹⁵C(O)R¹⁶, -NR¹⁵SO₂R¹⁶,
-SO₂NR¹⁵R¹⁶, -NR¹⁵R¹⁶, -OC(O)R¹⁷ or OC(O)NR¹⁵R¹⁶,
and R¹² represents hydrogen, halogen or C₁₋₄ alkyl;
or R¹¹ represents -NHR¹⁸ and R¹² and -NHR¹⁸ together form a 5- or 6- membered heterocyclic ring;
R¹³ represents hydrogen, halogen, -OR¹⁵ or -NR¹⁵R¹⁶;
R¹⁴ represents hydrogen, halogen, haloC₁₋₄ alkyl, -OR¹⁵, -NR¹⁵ R¹⁶, -OC(O)R¹⁷ or OC(O)NR¹⁵R¹⁶;
R¹⁵ and R¹⁶ each independently represents hydrogen or C₁₋₄ alkyl, or in the groups -NR¹⁵R¹⁶, -SO₂NR¹⁵R¹⁶ and -OC(O)NR¹⁵R¹⁶, R¹⁵ and R¹⁶ independently represent hydrogen or C₁₋₄ alkyl or together with the nitrogen atom to which they are attached form a 5-, 6- or 7- membered nitrogen-containing ring,
R¹⁷ represents an aryl group which may be unsubstituted or substituted by one or more substituents selected from halogen, C₁₋₄ alkyl,
hydroxy, C₁₋₄ alkoxy or halo C₁₋₄ alkyl; and
n is zero or an integer from 1 to 4;
provided that in the group (a), when R¹¹ represents -(CH₂)ₙOR¹⁵ and n is 1, R¹³ is not OH;
with the proviso that when one of R⁴ and R⁵ is hydrogen and the other is hydrogen or methyl, k is 1 or 2, Z is oxygen, the moiety -Ph(R¹)(R²)(R³) is phenyl, p-fluorophenyl or p-phenoxyphenyl then ring (a) is not phenyl monosubstituted in the m-position by bromine, chlorine or fluorine, or unsubstituted phenyl.

2. A compound according to claim 1 wherein Ar¹ is selected from group (a) or group (b), wherein the groups (a) and (b) are selected from the following groups, (i) to (xx): 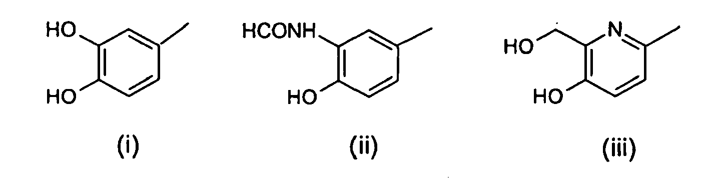 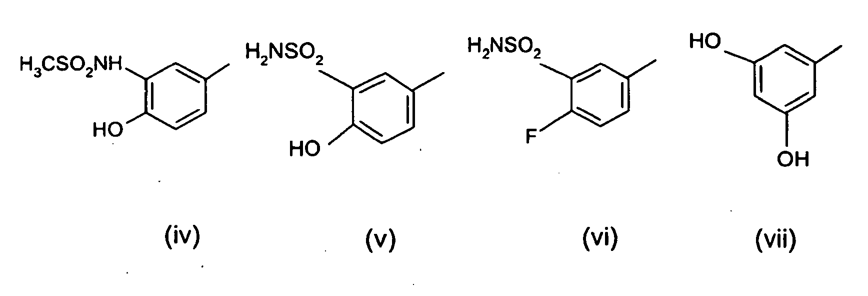 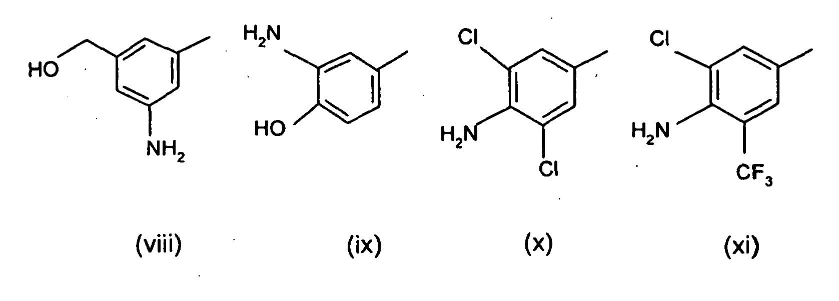 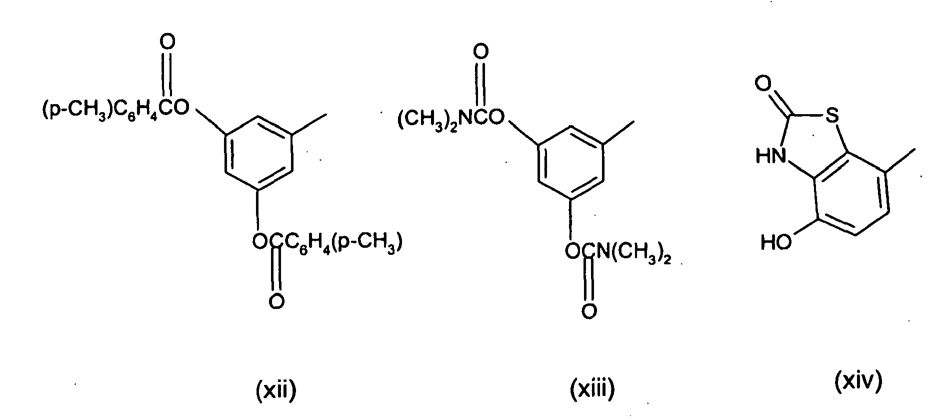 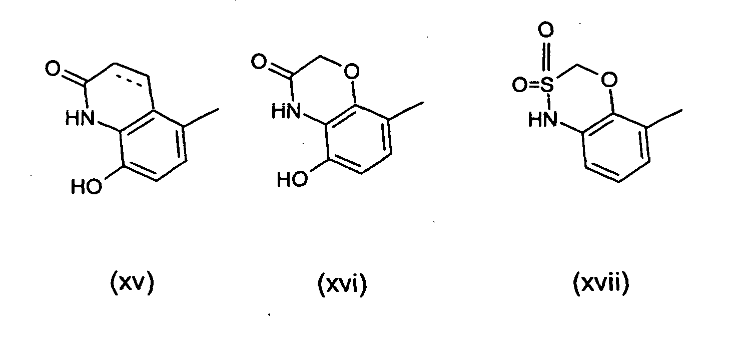 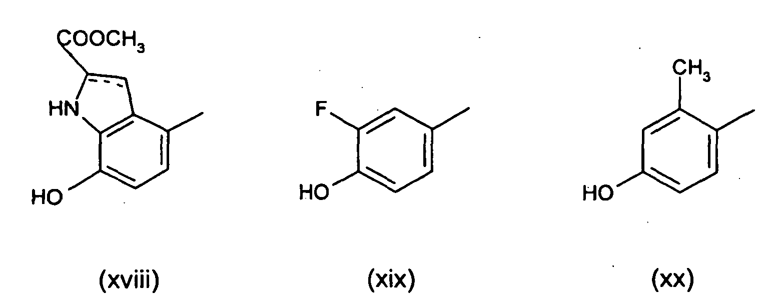 wherein the dotted line in (xv) and (xviii) denotes an optional double bond.

3. A compound of formula (I) according to claim 2 wherein group (a) is selected from a group of formula (iv) or (xix):

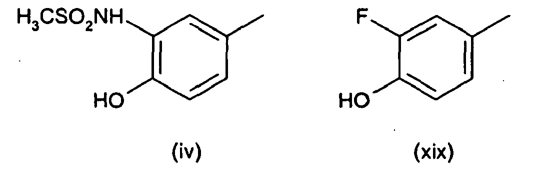

4. A compound of formula (I) according to claim 2 wherein group (b) is a group of formula (iii):

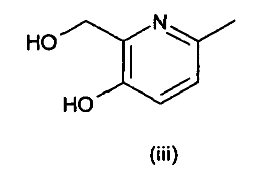

5. A compound of formula (I) according to any of claims 1-4 wherein R¹ is selected from hydrogen, C₁₋₄alkyl, hydroxy, cyano, C₁₋₆alkoxy, halo, XCO₂R⁸, XNR⁶COR⁷, XCONR⁷R⁸, -NR⁶C(O)NR⁷R⁸, XSOR⁶, XNR⁶SO₂NR⁷R⁸, XNR⁶SO₂NR⁷CO₂R⁷ and -NR⁶SO₂R⁷
wherein R⁶ and R⁷ are as defined above.

6. A compound of formula (I) according to claim 5 wherein R¹ is selected from XC(O)NR⁷R⁸ or hydrogen.

7. A compound of formula (I) according to any of claims 1-6 wherein R² and R³ each represent hydrogen.

8. A compound of formula (I) according to any of claims 1-7 wherein R⁴ and R⁵ each represent hydrogen.

9. A compound of formula (I) according to claim 1 which is selected from:
3-{[2-(4-{2-[((2*R*)-2-hydroxy-2-{4-hydroxy-3-[(methylsulfonyl)amino]phenyl}ethyl)amino]ethyl}phenoxy)ethoxy]methyl}benzamide;
*N*-{2-hydroxy-5-[(1*R*)-1-hydroxy-2-({2-[4-(4-phenylbutoxy)phenyl]ethyl}amino)ethyl]phenyl}methanesulfonamide;
*N*-(5-{(1*R*)-2-[(2-{4-[2-(benzyloxy)ethoxy]phenyl}ethyl)amino]-1-hydroxyethyl}-2-hydroxyphenyl)methanesulfonamide;
3-({2-[4-(2-{[(2*R*)-2-(3-fluoro-4-hydroxyphenyl)-2-hydroxyethyl]amino}ethyl)phenoxy]ethoxy}methyl)benzamide;
4-{(1*R*)-2-[(2-{4-[2-(benzyloxy)ethoxy]phenyl}ethyl)amino]-1-hydroxyethyl}-2-fluorophenol;
2-fluoro-4-({1*R*)-1-hydroxy-2-({2-[4-(4-phenylbutoxy)phenyl]ethyl}amino)ethyl]phenol; 3-[(2-{4-[2-({2-hydroxy-2-[5-hydroxy-6-(hydroxymethyl)pyridin-2-yl]ethyl}amino)ethyl]phenoxy}ethoxy)methyl]benzamide;
6-{2-[(2-{4-[2-(benzyloxy)ethoxy]phenyl}ethyl)amino]-1-hydroxyethyl}-2-(hydroxymethyl)pyridin-3-ol;
2-(hydroxymethyl)-6-[1-hydroxy-2-({2-[4-(4-phenylbutoxy)phenyl]ethyl}amino)ethyl]pyridin-3-ol;
and salts and solvates thereof.

10. A compound of formula (I), according to any of claims 1-9, or a pharmaceutically acceptable salt or solvate thereof for use in medical therapy.

11. A compound of formula (I), according to any of claims 1-9, or a pharmaceutically acceptable salt or solvate thereof for use in prophylaxis or treatment of a condition for which a selective β₂-adrenoreceptor agonist is indicated.

12. A pharmaceutical formulation comprising a compound of formula (I), according to any of claims 1-9, or a pharmaceutically acceptable salt or solvate thereof, and a pharmaceutically acceptable carrier or excipient, and optionally one or more other therapeutic ingredients.

13. The use of a compound of formula (I), according to any of claims 1-9, or a pharmaceutically acceptable salt or solvate thereof in the manufacture of a medicament for the prophylaxis or treatment of a clinical condition for which a selective β₂-adrenoreceptor agonist is indicated.

14. A process for the preparation of a compound of formula (I), according to any of claims 1-9, or a salt or solvate thereof, which comprises:
(a) deprotection of a protected intermediate, for example of formula (II): 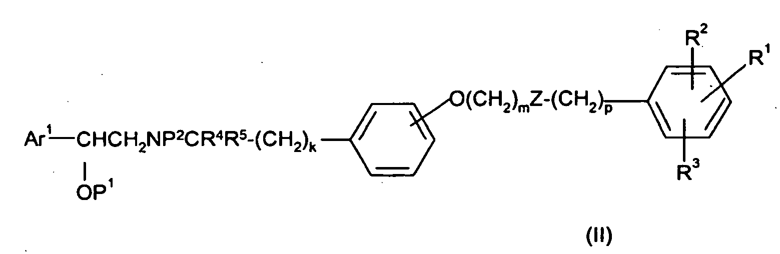 or a salt or solvate thereof, wherein Ar¹, R¹, R², R³, R⁴, R⁵, Z, k, m, and p are as defined in claim 1, and P¹ and P² are each independently either hydrogen or a protecting group provided that at least one of P¹ and P² is a protecting group; or
(b) alkylation of an amine of formula (X) 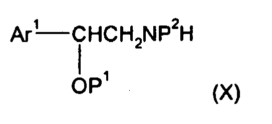 wherein Ar¹ is as defined in claim 1 and P¹ and P² are each independently either hydrogen or a protecting group,
with a compound of formula (XI): 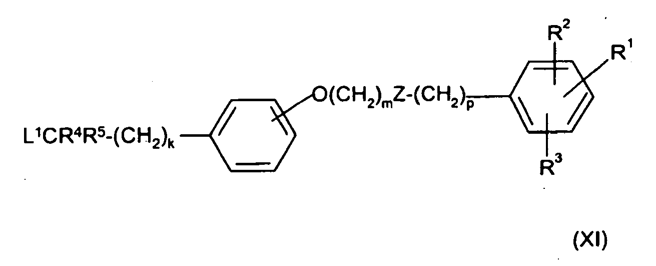 wherein R¹, R², R³, R⁴, R⁵, Z, k, m, and p are as defined in claim 1 and L¹ is a leaving group; or
(c) reacting a compound of formula (XII): 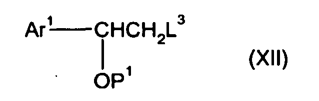 wherein Ar¹ is as defined in claim 1, P¹ is either hydrogen or a protecting group and L³ is a leaving group, with an amine of formula (XIII): 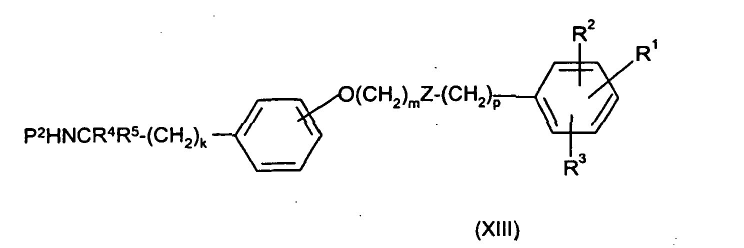 wherein R¹, R², R³, R⁴, R⁵, Z, k, m and p are as defined in claim 1 and P² is either hydrogen or a protecting group; or
d) reacting a compound of formula (X): 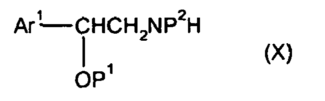 wherein Ar¹ is as defined in claim 1 and P¹ and P² are each independently either hydrogen or a protecting group,
with a compound of formula (XIV): 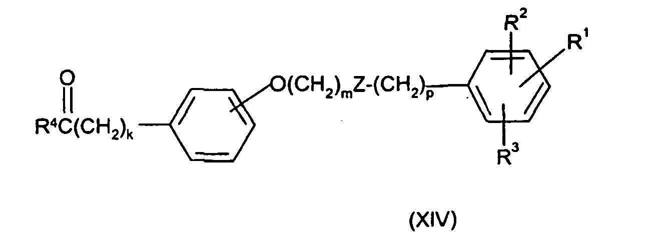 wherein R¹, R², R³, R⁴, Z, k, m and p are as defined in claim 1
under conditions suitable to effect reductive amination.
followed by the following steps in any order:
(i) optional removal of any protecting groups;
(ii) optional separation of an enantiomer from a mixture of enantiomers;
(iii) optional conversion of the product to a corresponding salt or solvate thereof.

## Patentansprüche

1. Eine Verbindung der Formel (I) 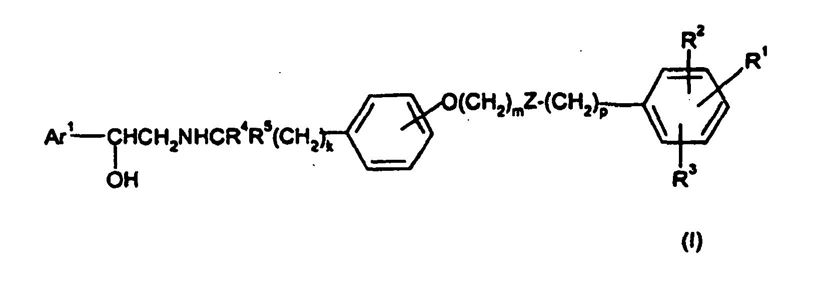 oder ein Salz oder Solvat davon, worin:
k eine ganze Zahl von 1 bis 3 ist;
m eine ganze Zahl von 2 bis 4 ist;
p eine ganze Zahl von 1 bis 4 ist;
Z O oder CH₂- ist;
R¹ ausgewählt ist aus Wasserstoff, C₁₋₆-Alkyl, Hydroxy, C₁₋₆-Alkoxy, Cyano, Nitro, Halogen, C₁₋₆-Halogenalkyl, XCO₂R⁸, -XC(O)NR⁷R⁸, -XNR⁶C(O)R⁷, -XNR⁶C(O)NR⁷R⁸, -XNR⁶C(O)NC(O)NR⁷R⁸, -XNR⁶SO₂R⁷, -XSO₂NR⁹R¹⁰, XSR⁶, XSOR⁶, XSO₂R⁶, XNR⁶SO₂NR⁷R⁸, XNR⁶SO₂NR⁷COOR⁷, -XNR⁷R⁸ und -XNR⁶C(O)OR⁷,
oder R¹ ist ausgewählt aus -X-Aryl, -X-Heteroaryl oder -X-(Aryloxy), wobei jedes gegebenenfalls mit 1 oder 2 Gruppen substituiert ist, die unabhängig ausgewählt sind aus Hydroxy, C₁₋₆-Alkoxy, Halogen, C₁₋₆-Alkyl, C₁₋₆-Halogenalkyl, -NR⁸C(O)R⁷, SR⁶, SOR⁶, -SO₂R⁶, -SO₂NR⁹R¹⁰, -CO₂R⁸, -NR⁷R⁸ oder Heteroaryl, das gegebenenfalls mit 1 oder 2 Gruppen substituiert ist, die unabhängig ausgewählt sind aus Hydroxy, C₁₋₆-Alkoxy, Halogen, C₁₋₆-Alkyl oder C₁₋₆-Halogenalkyl;
X -(CH₂)_{q}- oder C₂₋₆-Alkenylen ist;
q eine ganze Zahl von 0 bis 6 ist;
R⁶ und R⁷ unabhängig ausgewählt sind aus Wasserstoff, C₁₋₆-Alkyl, C₃₋₇-Cycloalkyl, Aryl, Heteroaryl, Heteroaryl(C₁₋₆-alkyl)- und Aryl(C₁₋₆-alkyl)-, und R⁶ und R⁷ jeweils unabhängig gegebenenfalls mit 1 oder 2 Gruppen substituiert sind, die unabhängig ausgewählt sind aus Halogen, C₁₋₆-Alkyl, C₃₋₇-Cycloalkyl, C₁₋₆-Alkoxy, C₁₋₆-Halogenalkyl, -NHC(O)(C₁₋₆-Alkyl), -SO₂(C₁₋₆-Alkyl), SO₂(Aryl), -CO₂H, -CO₂(C₁₋₄-Alkyl), -NH₂, -NH(C₁₋₆-Alkyl), Aryl(C₁₋₆-alkyl)-, Aryl(C₂₋₆-Alkenyl)-, Aryl(C₂₋₆-Alkinyl)-, Heteroaryl(C₁₋₆-alkyl)-, NHSO₂Aryl, -NH(Heteroaryl-C₁₋₆-alkyl), -NHSO₂Heteroaryl, -NHSO₂(C₁₋₆-Alkyl), -NHC(O)Aryl oder -NHC(O)Heteroaryl;
R⁸ ausgewählt ist aus Wasserstoff, C₁₋₆-Alkyl und C₃₋₇-Cycloalkyl;
oder R⁷ und R⁸ zusammen mit dem Stickstoffatom, an das sie gebunden sind, einen stickstoffhaltigen Ring mit 5, 6 oder 7 Gliedern bilden;
R⁹ und R¹⁰ unabhängig ausgewählt sind aus Wasserstoff, C₁₋₆-Alkyl, C₃₋₇-Cycloalkyl, Aryl, Heteroaryl, Heteroaryl(C₁₋₆-alkyl)- und Aryl(C₁₋₆-alkyl)-, oder R⁹ und R¹⁰ zusammen mit dem Stickstoff, an den sie gebunden sind, einen stickstoffhaltigen Ring mit 5, 6 oder 7 Gliedern bilden;
und R⁹ und R¹⁰ jeweils unabhängig mit einer oder zwei Gruppen substituiert ist, die unabhängig ausgewählt sind aus Halogen, C₁₋₆-Alkyl, C₃₋₇-Cycloalkyl und C₁₋₆-Halogenalkyl;
R² ausgewählt ist aus Wasserstoff, Hydroxy, C₁₋₆-Alkyl, C₁₋₆-Alkoxy, Halogen, Aryl, Aryl(C₁₋₆-alkyl)-, C₁₋₆-Halogenalkoxy und C₁₋₆-Halogenalkyl;
R³ ausgewählt ist aus Wasserstoff, Hydroxy, C₁₋₆-Alkyl, C₁₋₆-Alkoxy, Halogen, Aryl, Aryl(C₁₋₆-alkyl)-, C₁₋₆-Halogenalkoxy und C₁₋₆-Halogenalkyl;
R⁴ und R⁵ unabhängig ausgewählt sind aus Wasserstoff und C₁₋₄-Alkyl, mit der Maßgabe, daß die Gesamtanzahl an Kohlenstoffatomen in R⁴ und R⁵ nicht mehr als 4 ist; und
Ar¹ eine Gruppe ist, die ausgewählt ist aus 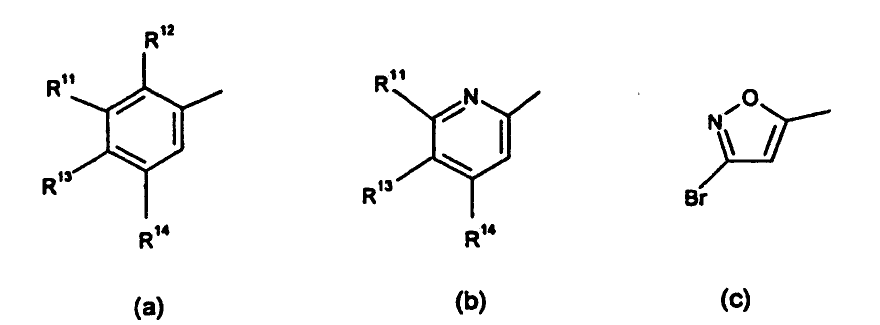 und 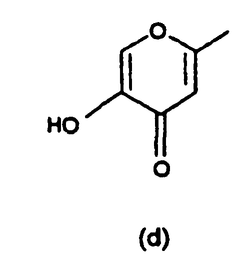
worin R¹¹ Halogen, -(CH₂)ₙOR¹⁵, -NR¹⁵C(O)R¹⁶, -NR¹⁵SO₂R¹⁶, -SO₂NR¹⁵R¹⁶, -NR¹⁵R¹⁶, -OC(O)R¹⁷ oder OC(O)NR¹⁵R¹⁶ darstellt,
und R¹² Wasserstoff, Halogen oder C₁₋₄-Alkyl darstellt;
oder R¹¹ stellt -NHR¹⁸ dar und R¹² und -NHR¹⁸ bilden zusammen einen Heterocyclyl-Ring mit 5 oder 6 Gliedern;
R¹³ Wasserstoff, Halogen, -OR¹⁵ oder -NR¹⁵R¹⁶ darstellt;
R¹⁴ Wasserstoff, Halogen, Halogen-C₁₋₄-alkyl, -OR¹⁵, -NR¹⁵R¹⁶, -OC(O)R¹⁷ oder OC(O)NR¹⁵R¹⁶ darstellt;
R¹⁵ und R¹⁶ jeweils unabhängig Wasserstoff oder C₁₋₄-Alkyl darstellt, oder in den Gruppen -NR¹⁵R¹⁶, -SO₂NR¹⁵R¹⁶ und -OC(O)NR¹⁵R¹⁶ stellen R¹⁵ und R¹⁶ unabhängig Wasserstoff oder C₁₋₄-Alkyl dar oder bilden zusammen mit dem Stickstoffatom, an das sie gebunden sind, einen stickstoffhaltigen Ring mit 5, 6 oder 7 Gliedern;
R¹⁷ eine Aryl-Gruppe darstellt, die unsubstituiert oder mit einem oder mehreren Substituenten substituiert sein kann, die ausgewählt sind aus Halogen, C₁₋₄-Alkyl, Hydroxy, C₁₋₄-Alkoxy oder Halogen-C₁₋₄-alkyl; und
n Null oder eine ganze Zahl von 1 bis 4 ist;
vorausgesetzt, daß in der Gruppe (a), wenn R¹¹ -(CH₂)ₙOR¹⁵ darstellt und n 1 ist, R¹³ nicht OH ist;
mit der Maßgabe, daß, wenn eins von R⁴ und R⁵ Wasserstoff und das andere Wasserstoff oder Methyl ist, k 1 oder 2 ist, Z Sauerstoff ist, die Einheit -Ph(R¹)(R²)(R³) Phenyl, p-Fluorphenyl oder p-Phenoxyphenyl ist, dann ist Ring (a) nicht Phenyl, das in der m-Stellung mit Brom, Chlor oder Fluor monosubstituiert ist, oder unsubstituiertes Phenyl.

2. Verbindung gemäß Anspruch 1, worin Ar¹ aus Gruppe (a) oder Gruppe (b) ausgewählt ist, worin die Gruppen (a) und (b) von den folgenden Gruppen (i) bis (xx) ausgewählt sind: 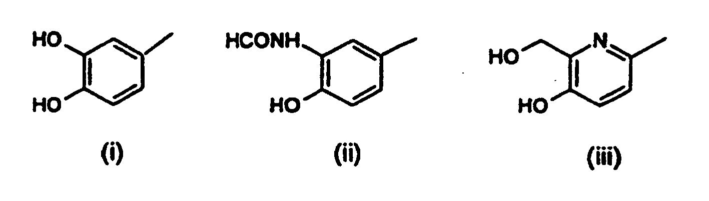 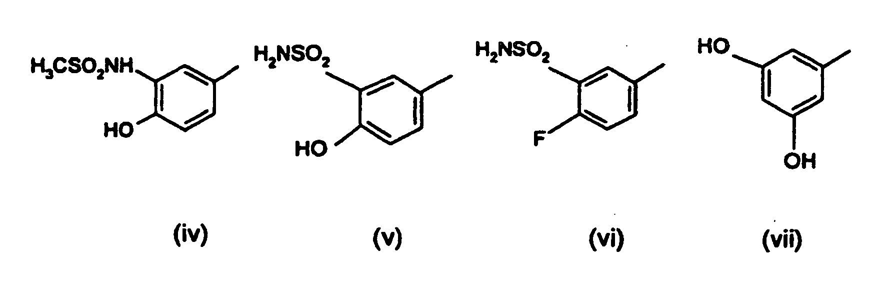 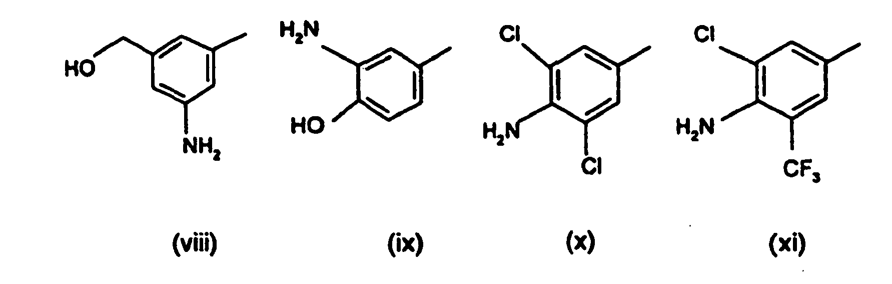 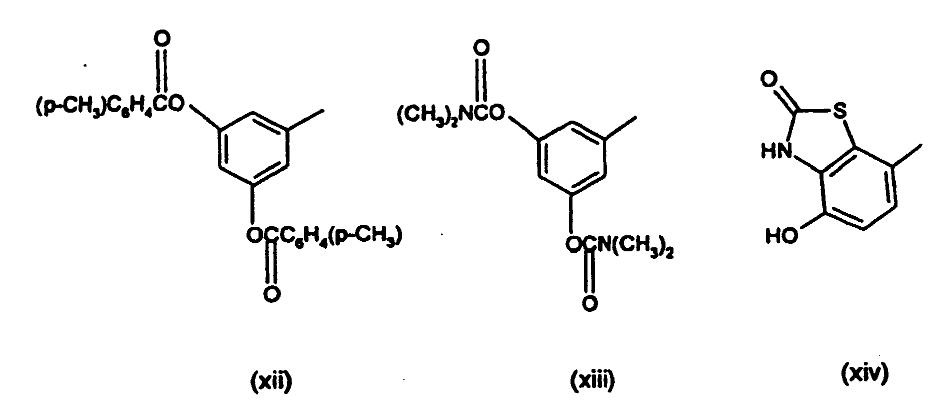 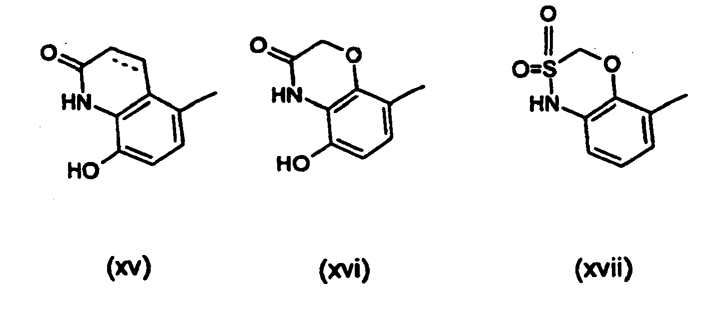 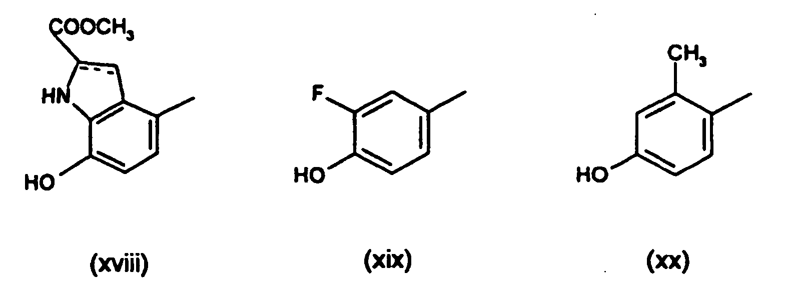 worin die gestrichelte Linie in (xv) und (xviii) eine optionale Doppelbindung anzeigt.

3. Verbindung der Formel (I) gemäß Anspruch 2, worin Gruppe (a) aus einer Gruppe der Formel (iv) oder (xix) ausgewählt ist:

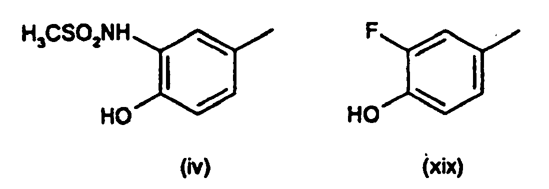

4. Verbindung der Formel (I) gemäß Anspruch 2, worin Gruppe (b) eine Gruppe der Formel (iii) ist:

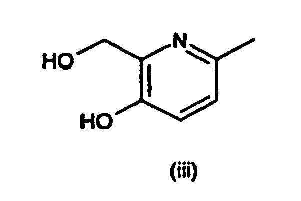

5. Verbindung der Formel (I) gemäß einem der Ansprüche 1 bis 4, worin R¹ ausgewählt ist aus Wasserstoff, C₁₋₄-Alkyl, Hydroxy, Cyano, C₁₋₆-Alkoxy, Halogen, XCO₂R⁸, XNR⁶COR⁷, XCONR⁷R⁸, -NR⁶C(O)NR⁷R⁸, XSOR⁶, XNR⁶SO₂NR⁷R⁸, XNR⁶SO₂NR⁷CO₂R⁷ und -NR⁶SO₂R⁷, worin R⁶ und R⁷ wie vorstehend definiert sind.

6. Verbindung der Formel (I) gemäß Anspruch 5, worin R¹ aus XC(O)NR⁷R⁸ oder Wasserstoff ausgewählt ist.

7. Verbindung der Formel (I) gemäß einem der Ansprüche 1 bis 6, worin R² und R³ jeweils Wasserstoff darstellt.

8. Verbindung der Formel (I) gemäß einem der Ansprüche 1 bis 7, worin R⁴ und R⁵ jeweils Wasserstoff darstellt.

9. Verbindung der Formel (I) gemäß Anspruch 1, die ausgewählt ist aus:
3-{[2-(4-{2-[((2R)-2-Hydroxy-2-{4-hydroxy-3-[(methylsulfonyl)amino]phenyl}ethyl)amino]ethyl}-phenoxy)ethoxy]methyllbenzamid;
N-{2-Hydroxy-5-[(1R)-1-Hydroxy-2-({2-[4-(4-phenylbutoxy)phenyl]ethyl}amino)ethyl]phenyl}-methansulfonamid;
N-(5-{(1R)-2-[(2-{4-[2-(Benzyloxy)ethoxy]-phenyl}ethyl)amino]-1-hydroxyethyl}-2-hydroxyphenyl)methansulfonamid;
3-({2-[4-(2-{[(2R)-2-(3-fluor-4-hydroxyphenyl)-2-hydroxyethyl]amino}ethyl)phenoxy]ethoxy}methyl)-benzamid;
4-{(1R)-2-[(2-{4-[2-(benzyloxy)ethoxy]phenyl}-ethyl)amino]-1-hydroxyethyll-2-fluorphenol;
2-Fluor-4-[(1R)-1-hydroxy-2-([2-[4-(4-phenylbutoxy)phenyl]ethyllamino)ethyl]phenol;
3-[(2-{4-[2-({2-Hydroxy-2-[5-hydroxy-6-(hydroxymethyl)pyridin-2-yl]ethyl}amino)ethyl]-phenoxylethoxy)methyl]benzamid;
6-{2-[(2-{4-[2-(Benzyloxy)ethoxy]phenyl}ethyl)-amino]-1-hydroxyethyll-2-(hydroxymethyl)pyridin-3-ol;
2-(Hydroxymethyl)-6-[1-hydroxy-2-({2-[4-(4-phenylbutoxy)phenyl]ethyl}amino)ethyl]pyridin-3-ol;
und Salze und Solvate davon.

10. Verbindung der Formel (I) gemäß einem der Ansprüche 1 bis 9 oder ein pharmazeutisch annehmbares Salz oder Solvat davon zur Verwendung in der medizinischen Therapie.

11. Verbindung der Formel (I) gemäß einem der Ansprüche 1 bis 9 oder ein pharmazeutisch annehmbares Salz oder Solvat davon zur Verwendung in der Prophylaxe oder Behandlung eines Zustands, für den ein selektiver β₂-Adrenorezeptoragonist angezeigt ist.

12. Pharmazeutische Formulierung, die eine Verbindung der Formel (I) gemäß einem der Ansprüche 1 bis 9 oder ein pharmazeutisch annehmbares Salz oder Solvat davon und einen pharmazeutisch annehmbaren Träger oder Exzipienten und gegebenenfalls einen oder mehrere andere therapeutische Bestandteile umfaßt.

13. Verwendung einer Verbindung der Formel (I) gemäß einem der Ansprüche 1 bis 9 oder eines pharmazeutisch annehmbaren Salzes oder Solvats davon in der Herstellung eines Medikaments für die Prophylaxe oder Behandlung eines klinischen Zustands, für den ein selektiver β₂-Adrenorezeptoragonist angezeigt ist.

14. Verfahren zur Herstellung einer Verbindung der Formel (I) gemäß einem der Ansprüche 1 bis 9 oder eines Salzes oder Solvats davon, das folgendes umfaßt:
(a) Entschützen einer geschützten Zwischenstufe, zum Beispiel eine der Formel (II): 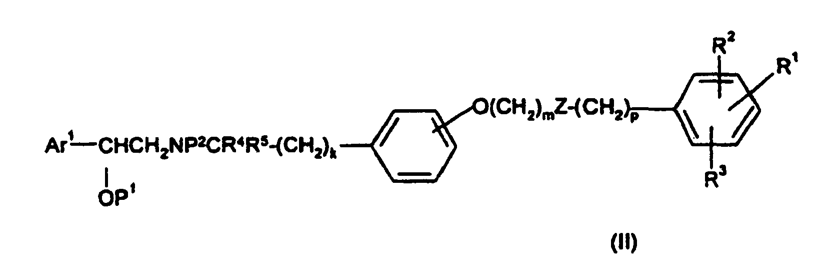 oder eines Salzes oder Solvats davon, worin Ar¹, R¹, R², R³, R⁴, R⁵, Z, k, m und p wie in Anspruch 1 definiert sind und P¹ und P² jeweils unabhängig entweder Wasserstoff oder eine Schutzgruppe ist, vorausgesetzt, daß wenigstens eins von P¹ und P² eine Schutzgruppe ist; oder
(b) Alkylierung eines Amins der Formel (X) 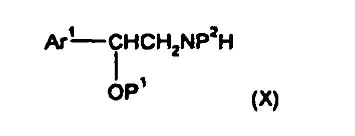 worin Ar¹ wie in Anspruch 1 definiert ist und P¹ und P² jeweils unabhängig entweder Wasserstoff oder eine Schutzgruppe ist,
mit einer Verbindung der Formel (XI): 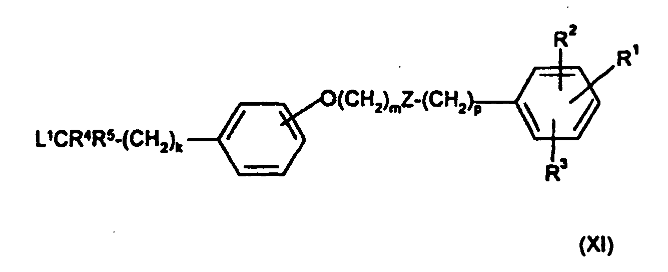 worin R¹, R², R³, R⁴, R⁵, Z, k, m und p wie in Anspruch 1 definiert sind, und L¹ eine Abgangsgruppe ist;
oder
(c) Umsetzen einer Verbindung der Formel (XII): 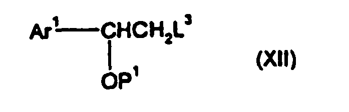 worin Ar¹ wie in Anspruch 1 definiert ist, P¹ entweder Wasserstoff oder eine Schutzgruppe ist und L³ eine Abgangsgruppe ist, mit einem Amin der Formel (XIII): 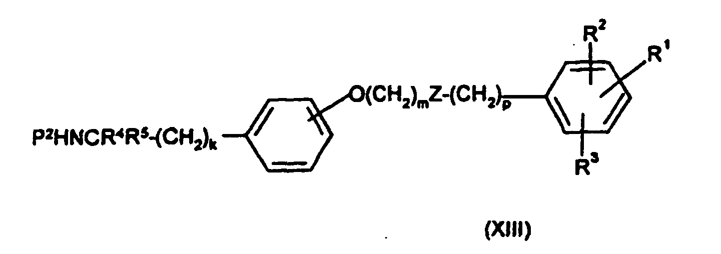 worin R¹, R², R³, R⁴, R⁵, Z, k, m und p wie in Anspruch 1 definiert sind und P² entweder Wasserstoff oder eine Schutzgruppe ist; oder
(d) Umsetzen einer Verbindung der Formel (X): 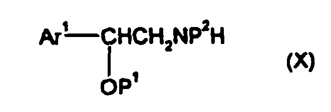 worin Ar¹ wie in Anspruch 1 definiert ist und P¹ und P² jeweils unabhängig entweder Wasserstoff oder eine Schutzgruppe ist,
mit einer Verbindung der Formel (XIV): 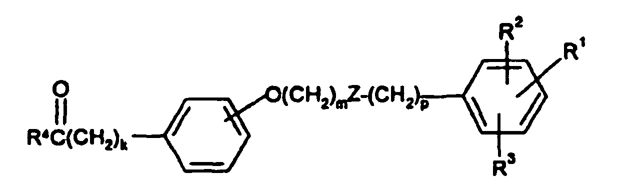 worin R¹, R², R³, R⁴, Z, k, m und p wie in Anspruch 1 definiert sind, unter Bedingungen, die zum Bewirken einer reduktiven Aminierung geeignet sind,
gefolgt von den folgenden Schritten in beliebiger Reihenfolge:
(i) optionales Entfernen irgendwelcher Schutzgruppen;
(ii) optionales Trennen eines Enantiomers von einem Gemisch aus Enantiomeren;
(iii) optionales Umwandeln des Produkts in ein entsprechendes Salz oder Solvat davon.

## Revendications

1. Composé de formule (I) 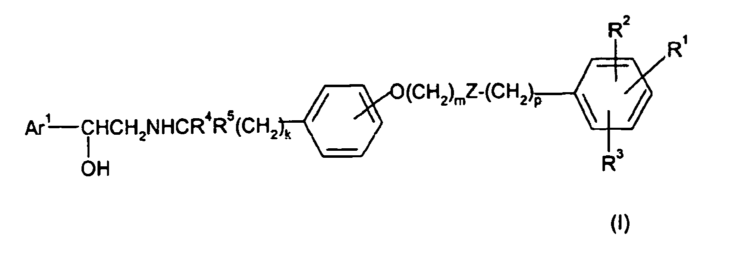 ou un sel ou un solvate de celui-ci, où :
k est un nombre entier de 1 à 3 ;
m est un nombre entier de 2 à 4 ;
p est un nombre entier de 1 à 4 ;
Z représente O ou CH₂-
R¹ est choisi parmi un atome d'hydrogène, un groupe alkyle en C₁ à C₆, hydroxy, alcoxy en C₁ à C₆, cyano, nitro, halogéno, halogénoalkyle en C₁ à C₆, XCO₂R⁸, -XC(O)NR⁷R⁸, -XNR⁶C(O)R⁷, -XNR⁶C(O)NR⁷R⁸, -XNR⁶C(O)NC(O)NR⁷R⁸, -XNR⁶SO₂R⁷, -XSO₂NR⁹R¹⁰, XSR⁶, XSOR⁶, XSO₂R⁶, XNR⁶SO₂NR⁷R⁸, XNR⁶SO₂NR⁷COOR⁷, -XNR⁷R⁸, -XNR⁶C(O)OR⁷,
ou R¹ est choisi parmi -X-aryle, -X-hétaryle ou -X-(aryloxy), chacun éventuellement substitué par 1 ou 2 groupes choisis indépendamment parmi un groupe hydroxy, alcoxy en C₁ à C₆, halogéno, alkyle en C₁ à C₆, halogénoalkyle en C₁ à C₆, -NR⁶C(O)R⁷, SR⁶, SOR⁶, -SO₂R⁶, -SO₂NR⁹R¹⁰, -CO₂R⁸, -NR⁷R⁸, ou hétaryle éventuellement substitué par 1 ou 2 groupes choisis indépendamment parmi les groupes hydroxy, alcoxy en C₁ à C₆, halogéno, alkyle en C₁ à C₆, ou halogénoalkyle en C₁ à C₆ ;
X représente -(CH₂)_{q}- ou un groupe alcénylène en C₂ à C₆ ;
q est un nombre entier de 0 à 6 ;
R⁶ et R⁷ sont choisis indépendamment parmi un atome d'hydrogène, un groupe alkyle en C₁ à C₆, cycloalkyle en C₃ à C₇, aryle, hétaryle, hétaryl(alkyl en C₁ à C₆) - et aryl(alkyl en C₁ à C₆)- et R⁶ et R⁷ sont chacun indépendamment éventuellement substitué par 1 ou 2 groupes choisis indépendamment choisis parmi les groupes halogéno, alkyle en C₁ à C₆, cycloalkyle en C₃ à C₇, alcoxy en C₁ à C₆, halogénoalkyle en C₁ à C₆, -NHC(O)(alkyle en C₁ à C₆), -SO₂(alkyle en C₁ à C₆), -SO₂(aryle), -CO₂H, -CO₂(alkyle en C₁ à C₄), -NH₂, -NH(alkyle en C₁ à C₆), aryl(alkyl en C₁ à C₆)-, aryl (alcényl en C₂ à C₆)-, aryl (alcynyl en C₂ à C₆)-, hétaryl(alkyl en C₁ à C₆)-, -NHSO₂aryle, -NH(hétarylalkyle en C₁ à C₆), -NHSO₂hétaryle, -NHSO₂ (alkyle en C₁ à C₆), -NHC(O)aryle ou -NHC(O)hétaryle ;
R⁸ est choisi parmi un atome d'hydrogène, un groupe alkyle en C₁ à C₆ et cycloalkyle en C₃ à C₇ ;
ou R⁷ et R⁸, conjointement avec l'atome d'azote auquel ils sont liés, forment un cycle contenant de l'azote de 5, 6 ou 7 chaînons ;
R⁹ et R¹⁰ sont choisis indépendamment parmi un atome d'hydrogène, un groupe alkyle en C₁ à C₆, cycloalkyle en C₃ à C₇, aryle, hétaryle, hétaryl (alkyl en C₁ à C₆) - et aryl(alkyl en C₁ à C₆) -, ou R⁹ et R¹⁰, conjointement avec l'atome d'azote auquel ils sont liés, forment un cycle contenant de l'azote de 5, 6 ou 7 chaînons ;
et R⁹ et R¹⁰ sont chacun éventuellement substitués par un ou deux groupes choisis indépendamment parmi les groupes halogéno, alkyle en C₁ à C₆, cycloalkyle en C₃ à C₇ et halogénoalkyle en C₁ à C₆ ;
R² est choisi parmi un atome d'hydrogène, un groupe hydroxy, alkyle en C₁ à C₆, alcoxy en C₁ à C₆, halogéno, aryle, aryl(alkyl en C₁ à C₆)-, halogénoalcoxy en C₁ à C₆ et halogénoalkyle en C₁ à C₆ ;
R³ est choisi parmi un atome d'hydrogène, un groupe hydroxy, alkyle en C₁ à C₆, alcoxy en C₁ à C₆, halogéno, aryle, aryl (alkyl en C₁ à C₆)-, halogénoalcoxy en C₁ à C₆ et halogénoalkyle en C₁ à C₆ ;
R⁴ et R⁵ sont choisis indépendamment parmi un atome d'hydrogène et un groupe alkyle en C₁ à C₄ à condition que le nombre total d'atomes de carbone dans R⁴ et R⁵ ne soit pas supérieur à 4 ; et
Ar¹ représente un groupe choisi parmi 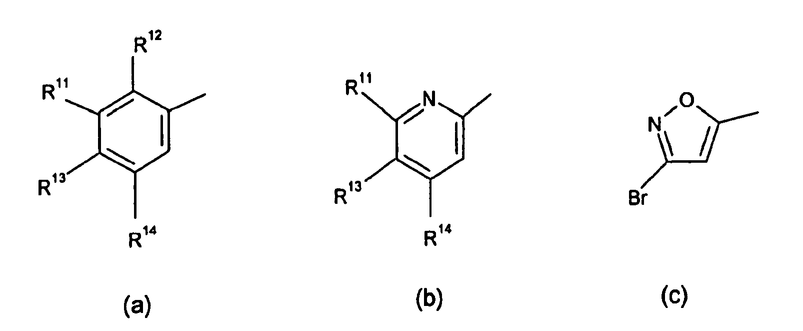 et 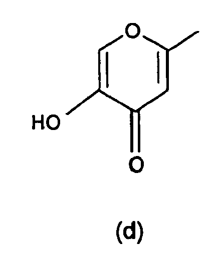
où R¹¹ représente un atome d'halogène, -(CH₂)ₙOR¹⁵, -NR¹⁵C(O)R¹⁶, -NR¹⁵SO₂R¹⁶, -SO₂NR¹⁵R¹⁶, -NR¹⁵R¹⁶, -OC(O)R¹⁷ ou OC(O)NR¹⁵R¹⁶,
et R¹² représente un atome d'hydrogène, d'halogène ou un groupe en C₁ à C₄ ;
ou R¹¹ représente -NHR¹⁸ et R¹² et -NHR¹⁸ ensemble forment un cycle hétérocyclique de 5 ou 6 chaînons ;
R¹³ représente un atome d'hydrogène, d'halogène, -OR¹⁵ ou -NR¹⁵R¹⁶ ;
R¹⁴ représente un atome d'hydrogène, d'halogène, un groupe halogénoalkyle en C₁ à C₄, -OR¹⁵, -NR¹⁵R¹⁶, -OC(O)R¹⁷ ou OC(O)NR¹⁵R¹⁶ ;
R¹⁵ et R¹⁶ chacun indépendamment représentent un atome d'hydrogène ou un groupe alkyle en C₁ à C₄, ou dans les groupes -NR¹⁵R¹⁶, -SO₂NR¹⁵R¹⁶ et -OC(O)NR¹⁵R¹⁶, R¹⁵ et R¹⁶ représentent indépendamment un atome d'hydrogène ou un groupe alkyle en C₁ à C₄ ou conjointement avec l'atome d'azote auquel ils sont fixés forment un cycle contenant de l'azote de 5, 6 ou 7 chaînons,
R¹⁷ représente un groupe aryle qui peut être non substitué ou substitué par un ou plusieurs substituants choisis parmi un atome d'halogène, un groupe alkyle en C₁ à C₄, hydroxy, alcoxy en C₁ à C₄ ou halogénoalkyle en C₁ à C₄ ; et
n vaut zéro ou est un nombre entier de 1 à 4 ;
à condition que dans le groupe (a), lorsque R¹¹ représente -(CH₂)ₙOR¹⁵ et n vaut 1, R¹³ ne représente pas OH ;
à condition que lorsque l'un de R⁴ et R⁵ représente un atome d'hydrogène et l'autre représente un atome d'hydrogène ou un groupe méthyle, k vaut 1 ou 2, Z représente un atome d'oxygène, le radical -Ph(R¹) (R²) (R³) représente un groupe phényle, p-fluorophényle ou p-phénoxyphényle, alors le cycle (a) ne représente pas un groupe phényle monosubstitué en position m par un atome de brome, de chlore ou de fluor, ou phényle non substitué.

2. Composé selon la revendication 1, dans lequel Ar¹ est choisi parmi le groupe (a) ou le groupe (b), où les groupes (a) et (b) sont choisis parmi les groupes suivants, (i) à (xx) : 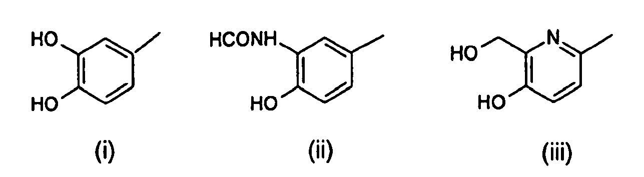 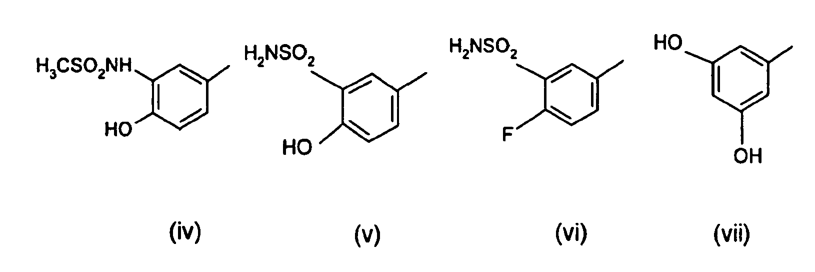 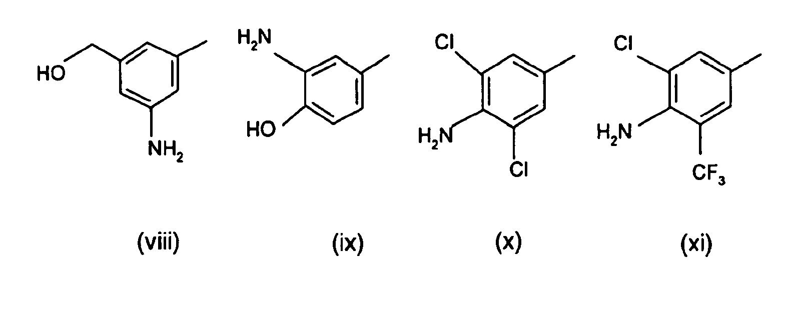 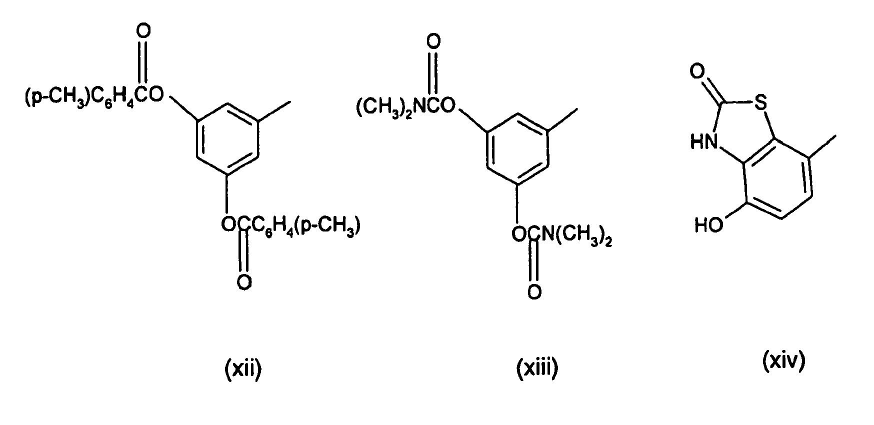 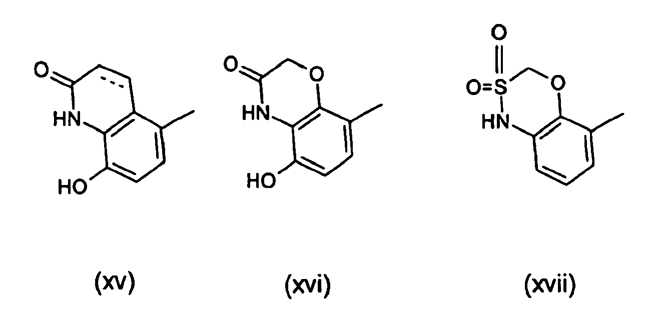 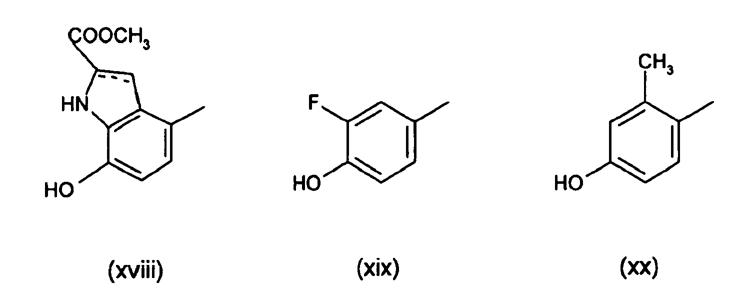 où la ligne en pointillé dans (xv) et (xviii) indique une éventuelle double liaison.

3. Composé de formule (I) selon la revendication 2, dans lequel le groupe (a) est choisi parmi un groupe de formule (iv) ou (xix) :

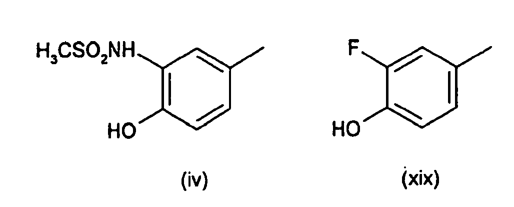

4. Composé de formule (I) selon la revendication 2, dans lequel le groupe (b) est un groupe de formule (iii) :

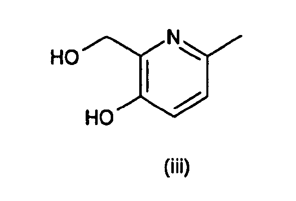

5. Composé de formule (I) selon l'une quelconque des revendications 1 à 4, dans lequel R¹ est choisi parmi un atome d'hydrogène, un groupe alkyle en C₁ à C₉, hydroxy, cyano, alcoxy en C₁ à C₆, halogéno, XCO₂R⁸, XNR⁶COR⁷, XCONR⁷R⁸, -NR⁶C(O)NR⁷R⁸, XSOR⁶, XNR⁶SO₂NR⁷R⁸, XNR⁶SO₂NR⁷CO₂R⁷ et -NR⁶SO₂R⁷ où R⁶ et R⁷ sont tels que définis ci-dessus.

6. Composé de formule (I) selon la revendication 5, dans lequel R¹ est choisi parmi XC(O)NR⁷R⁸ ou un atome d'hydrogène.

7. Composé de formule (I) selon l'une quelconque des revendications 1 à 6, dans lequel R² et R³ représentent chacun un atome d'hydrogène.

8. Composé de formule (I) selon l'une quelconque des revendications 1 à 7, dans lequel R⁴ et R⁵ représentent chacun un atome d'hydrogène.

9. Composé de formule (I) selon la revendication 1 qui est choisi parmi :
le 3-{[2-(4-(2-[((2*R*)-2-hydroxy-2-{4-hydroxy-3-[(méthyl-sulfonyl)amino]phényl}éthyl)amino]éthyl}phénoxy)éthoxy]-méthyl}benzamide ;
le *N*-{2-hydroxy-5-[(1*R*)-1-hydroxy-2-({2-[4-(4-phényl-butoxy)phényl]éthyl}amino)éthyl]phényl}méthanesulfonamide ;
le *N*-(5-{(1*R*)-2-[(2-(4-[2-(benzyloxy)éthoxy]phényl}-éthyl)amino]-1-hydroxyéthyl}-2-hydroxyphényl)méthane-sulfonamide ;
le 3-({2-[4-(2-{[(2*R*)-2-(3-fluoro-4-hydroxyphényl)-2-hydroxyéthyl]amino}éthyl)phénoxy]éthoxy}méthyl)benzamide ;
le 4-{(1*R*)-2-[(2-{4-[2-(benzyloxy)éthoxy]phényl}éthyl)-aminol-1-hydroxyéthyl}-2-fluorophénol ;
le 2-fluoro-4-[(1*R*)-1-hydroxy-2-({2-[4-(4-phénylbutoxy)-phényl]éthyl}amino)éthyl]phénol ;
le 3-[(2-{4-[2-({2-hydroxy-2-[5-hydroxy-6-(hydroxyméthyl)pyridin-2-yl]éthyl}amino)éthyl]phénoxy}éthoxy)méthyl]-benzamide ;
le 6-{2-[(2-{4-[2-(benzyloxy)éthoxy]phényl}éthyl)amino]-1-hydroxyéthyl}-2-(hydroxyméthyl)pyridin-3-ol ;
le 2-(hydroxyméthyl)-6-[1-hydroxy-2-({2-[4-(4-phénylbutoxy)phényl]éthyl}amino)éthyl]pyridin-3-ol ;
et des sels et des solvates de ceux-ci.

10. Composé de formule (I), selon l'une quelconque des revendications 1 à 9, ou un sel ou un solvate pharmaceutiquement acceptable de celui-ci pour une utilisation en thérapie médicale.

11. Composé de formule (I), selon l'une quelconque des revendications 1 à 9, ou un sel ou un solvate pharmaceutiquement acceptable de celui-ci pour une utilisation dans la prophylaxie ou le traitement d'une affection pour laquelle un agoniste de récepteur β₂-adrénergique sélectif est indiqué.

12. Formulation pharmaceutique comprenant un composé de formule (I), selon l'une quelconque des revendications 1 à 9, ou un sel ou un solvate pharmaceutiquement acceptable de celui-ci, et un support ou un excipient pharmaceutiquement acceptable, et éventuellement un ou plusieurs autres composants thérapeutiques.

13. Utilisation d'un composé de formule (I), selon l'une quelconque des revendications 1 à 9, ou d'un sel ou d'un solvate pharmaceutiquement acceptable de celui-ci, dans la fabrication d'un médicament destiné à la prophylaxie ou au traitement d'une affection clinique pour laquelle un agoniste de récepteur β₂-adrénergique sélectif est indiqué.

14. Procédé de préparation d'un composé de formule (I), selon l'une quelconque des revendications 1 à 9, ou d'un sel ou d'un solvate de celui-ci, qui comprend :
(a) la déprotection d'un intermédiaire protégé, par exemple de formule (II) : 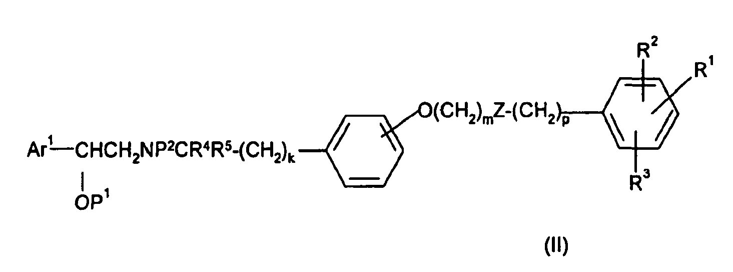 ou d'un sel ou d'un solvate de celui-ci, où Ar¹, R¹, R², R³, R⁴, R⁵, Z, k, m et p sont tels que définis dans la revendication 1, et P¹ et P² représentent chacun indépendamment soit un atome d'hydrogène soit un groupe protecteur à condition qu'au moins l'un de P¹ et P² soit un groupe protecteur ; ou
(b) l'alkylation d'une amine de formule (X) 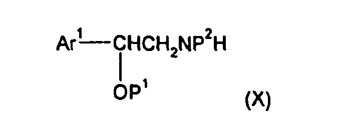 où Ar¹ est tel que défini dans la revendication 1 et P¹ et P² représentent chacun indépendamment soit un atome d'hydrogène soit un groupe protecteur,
avec un composé de formule (XI) : 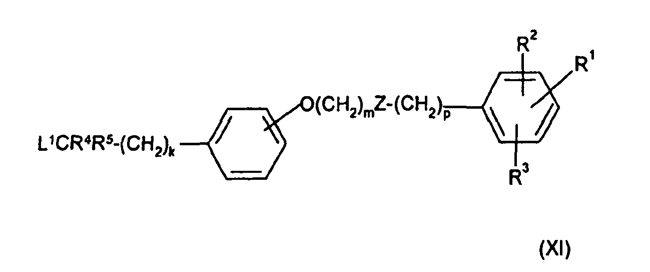 où R¹, R², R³, R⁴, R⁵, Z, k, m et p sont tels que définis dans la revendication 1 et L¹ représente un groupe partant ;
ou
(c) la réaction d'un composé de formule (XII) : 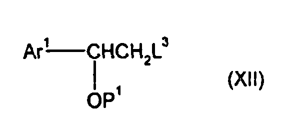 où Ar¹ est tel que défini dans la revendication 1, P¹ représente soit un atome d'hydrogène soit un groupe protecteur et L³ représente un groupe libérable, avec une amine de formule (XIII) : 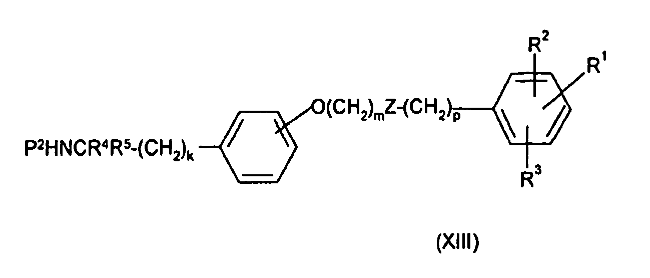 où R¹, R², R³, R⁴, R⁵, Z, k, m et p sont tels que définis dans la revendication 1 et P² représente soit un atome d'hydrogène soit un groupe protecteur ; ou
d) la réaction d'un composé de formule (X) : 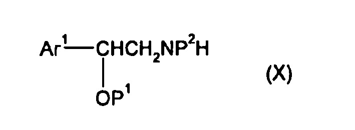 où Ar¹ est tel que défini dans la revendication 1 et P¹ et P² représentent chacun indépendamment soit un atome d'hydrogène soit un groupe protecteur,
avec un composé de formule (XIV) : 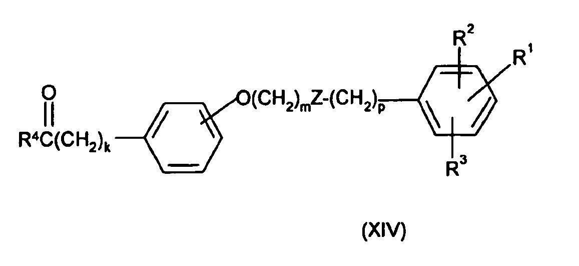 où R¹, R², R³, R⁴, R⁵, Z, k, m et p sont tels que définis dans la revendication 1
dans des conditions appropriées pour effectuer une amination réductrice,
suivie par les étapes suivantes dans n'importe quel ordre :
(i) une élimination éventuelle de groupes protecteurs quelconques ;
(ii) une séparation éventuelle d'un énantiomère à partir d'un mélange d'énantiomères ;
(iii) une conversion éventuelle du produit en un sel ou un solvate correspondant de celui-ci.
